(19) Europäisches Patentamt
European Patent Office
Office européen des brevets

(11) **EP 4 692 082 A1**

(12) **EUROPEAN PATENT APPLICATION**
published in accordance with Art. 153(4) EPC


(43) Date of publication:
**11.02.2026 Bulletin 2026/07**

(21) Application number: **24777936.6**

(22) Date of filing: **22.03.2024**

(51) International Patent Classification (IPC):
***C07D 413/14*** *(2006.01)* ***A61K 31/4545*** *(2006.01)*
***A61K 31/496*** *(2006.01)* ***A61P 35/00*** *(2006.01)*

(52) Cooperative Patent Classification (CPC):
**A61K 31/4545; A61K 31/496; A61P 35/00;**
**C07D 413/14**

(86) International application number:
**PCT/CN2024/083385**

(87) International publication number:
**WO 2024/199147 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **24.03.2023 CN 202310293642**



(71) Applicant: **Zhangzhou Pien Tze Huang Pharmaceutical Co., Ltd.**
**Fujian Province, 363000 (CN)**

(72) Inventors:
- **ZHUANG, Yichao**
  **Zhangzhou, Fujian 363000 (CN)**
- **LAN, Wenliang**
  **Zhangzhou, Fujian 363000 (CN)**
- **ZENG, Lei**
  **Zhangzhou, Fujian 363000 (CN)**
- **GAO, Longhui**
  **Zhangzhou, Fujian 363000 (CN)**
- **HUANG, Jing**
  **Zhangzhou, Fujian 363000 (CN)**
- **LUO, Yunfu**
  **Shanghai 200131 (CN)**
- **XU, Yu**
  **Shanghai 200131 (CN)**

(74) Representative: **Dehns**
**10 Old Bailey**
**London EC4M 7NG (GB)**


(54) **CRYSTAL FORM OF 2,6-PIPERIDINEDIONE COMPOUND, AND PREPARATION METHOD THEREFOR AND USE THEREOF**


(57) Disclosed in the present invention are a crystal form of a 2,6-piperidinedione compound, and a preparation method therefor and the use of the crystal form in the preparation of a drug for treating related diseases. Specifically disclosed are a crystal form A of the compound of formula (I) and a preparation method therefor.

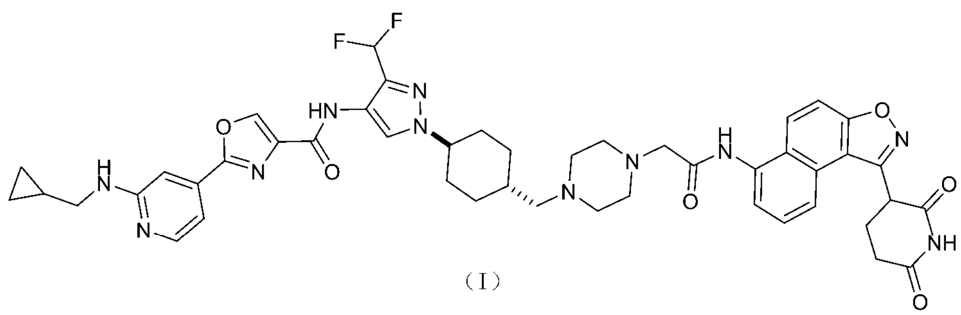

(I)


EP 4 692 082 A1

## Description

The present application claims the following priority:

The present application claims the priority and right of the Chinese patent application No. 2023102936424 filed with the China National Intellectual Property Administration on March 24, 2023. The contents of the Chinese patent application are incorporated herein by reference in its entirety.

TECHNICAL FIELD

The present disclosure relates to a crystal form of a 2,6-piperidinedione compound, a preparation method therefor, and a use thereof, further including a use of the crystal form in the manufacture of a medicament for treating related diseases. Specifically, the present disclosure relates to a crystal form A of a compound of formula (I) and a preparation method therefor.

BACKGROUND

Interleukin-1 receptor-associated kinase 4 (IRAK4) plays a pivotal role in the signal transduction pathways of the Toll-like receptor (TLR) family and the interleukin-1 receptor (IL-1R) family, receiving upstream signals to activate downstream JNK and NF-κB signaling pathways. IRAK4 is closely associated with the development and progression of human inflammatory immune diseases and tumors.

The Toll-like receptor (TLR) signaling transduction protein myeloid differentiation factor (MyD88) is frequently mutated in various lymphomas, such as Waldenström macroglobulinemia, lymphoplasmacytic lymphoma, immune-resistant large B-cell lymphoma, and marginal zone lymphoma, with mutation rates of 95-97%, 79%, 50-80%, 15-29%, and 6-10%, respectively. IRAK4 is involved in nearly all biological functions of MyD88, making it a highly attractive and promising drug target, particularly for the treatment of MyD88-driven lymphomas.

Research has found that IRAK4 not only phosphorylates proteins but also forms complexes with MyD88 to exert its biological functions. The activation of the JNK signaling pathway by IRAK4 requires its phosphorylation function, whereas the activation of the NF-κB signaling pathway does not, indicating that IRAK4 possesses both protein kinase and scaffolding protein functions and plays a role in signaling pathways. Therefore, conventional small molecule kinase inhibitors targeting IRAK4 cannot completely block all biological functions of IRAK4.

Proteolysis targeting chimera (PROTAC) is a technique that applies the ubiquitin-proteasome system to target specific proteins and induce their intracellular degradation. The ubiquitin-proteasome system is the main pathway for intracellular protein degradation, mainly responsible for the removal of denatured, mutated, or harmful proteins from the cell as its normal physiological functions. 80% or more of intracellular protein degradation is dependent on the ubiquitin-proteasome system. PROTAC utilizes the cell's own protein destruction mechanism to remove specific targeted proteins from the cell. To date, PROTAC technology has become increasingly mature and can be used to target a variety of proteins, including scaffold proteins, transcription factors, enzymes, and regulatory proteins. Additionally, domide drugs are referred to as immunomodulatory drugs (IMiDs), which activate the E3 ubiquitin ligase complex formed with Cereblon (CRBN) to ubiquitinate transcription factors IKZF1 (zinc finger transcription factor 1) and IKZF3 (zinc finger transcription factor 3), subsequently recognized and degraded by the proteasome, thereby exerting cytotoxic effects on tumors. CRBN, as an important target for antitumor and immunomodulatory drugs, has been demonstrated to exhibit definitive therapeutic efficacy in various hematologic malignancies, skin diseases such as erythema nodosum leprosum, and autoimmune diseases such as systemic lupus erythematosus.

Therefore, the development of PROTAC and IMiD bifunctional molecules targeting IRAK4, by degrading IRAK4 and removing IRAK4, more thoroughly blocks all functions of IRAK4, thereby fundamentally achieving comprehensive inhibition of the IRAK4 signaling pathway, while possessing good CRBN modulatory effects to exert synergistic therapeutic actions, thus better exerting antitumor effects, enhancing clinical therapeutic effects.

SUMMARY

In one aspect, the present disclosure provides a crystal form A of a compound of formula (I),

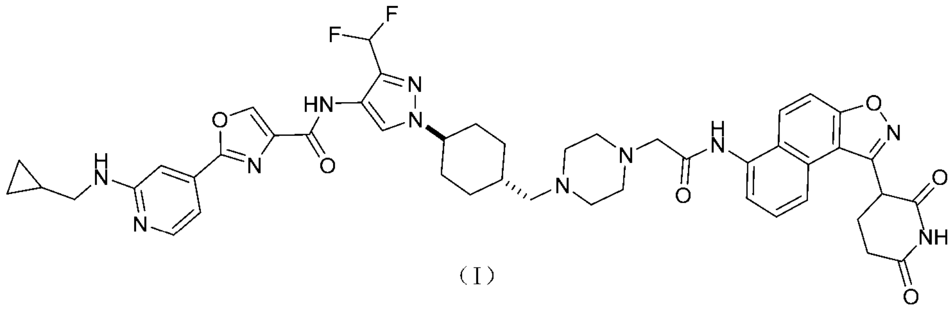

(I)

wherein the crystal form A of the compound of formula (I) has an X-ray powder diffraction pattern comprising characteristic diffraction peaks at the following 2θ angles: 8.91±0.20°, 11.15±0.20°, 17.26±0.20°, 21.14±0.20°, and 23.38±0.20°.

In some embodiments of the present disclosure, the X-ray powder diffraction pattern of the crystal form A of the compound of formula (I), expressed in 20 angles, comprises at least 6, 7, or 8 diffraction peaks selected from the group consisting of: 5.02±0.20°, 8.91±0.20°, 11.15±0.20°, 15.08±0.20°, 17.26±0.20°,18.38±0.20°, 21.14±0.20°, 23.3810.20°, and 23.57±0.20°.

In some embodiments of the present disclosure, the X-ray powder diffraction pattern of the crystal form A of the compound of formula (I) comprises characteristic diffraction peaks at the following 20 angles: 5.02±0.20°, 8.91±0.20°, 11.15+0.20°, 15.08±0.20°, 17.26±0.20°,18.38±0.20°, 21.1410.20°, and 23.57±0.20°.

In some embodiments of the present disclosure, the X-ray powder diffraction pattern of the crystal form A of the compound of formula (I), expressed in 20 angles, comprises at least 10, 11, 12, or 13 diffraction peaks selected from the group consisting of: 5.02±0.20°, 8.91±0.20°, 9.80±0.20°, 11.15+0.20°, 13.87±0.20°, 14.70±0.20°, 15.08±0.20°, 17.26 ±0.20°,18.38±0.20° 21.1410.20°, 21.5710.20°, 23.10±.20°, 23.38±.20°, and 23.574±.20°.

In some embodiments of the present disclosure, the X-ray powder diffraction pattern of the crystal form A of the compound of formula (I) comprises characteristic diffraction peaks at the following 20 angles: 5.02±0.20°, 8.91±0.20°, 9.80±.20°, 11.15±0.20°, 13.87±0.20°, 14.70±0.20°, 15.08±0.20°, 17.26±0.20°,18.38±0.20° 21.14±0.20°, 21.57 ±0.20°, and 23.38±0.20°.

In some embodiments of the present disclosure, the X-ray powder diffraction pattern of the crystal form A of the compound of formula (I) comprises characteristic diffraction peaks at the following 20 angles: 5.02±0.20° 8.91±0.20°, 9.80±.20°, 11.15+0.20°, 12.72±.20°, 13.87±.20°, 14.70±0.20°, 15.08±0.20°, 16.41±.20°, 17.26±0.20°,18.38±0.20°, 19.22±0.20°, 19.86±0.20°, 21.14±0.20°, 21.57±0.20°, 22.29±0.20°, 23.10±0.20°, 23.38±.20°, 23.57±0.200°, 25.71 ±0.20°, 26.49±0.20°, and 28.91±0.20°.

In some embodiments of the present disclosure, the X-ray powder diffraction pattern of the crystal form A of the compound of formula (I) comprises characteristic diffraction peaks at the following 20 angles: 5.02°, 8.91°, 9.80°, 11.15°, 12.72°, 13.87°, 14.70°, 15.08°, 16.41°, 17.26°, 18.38°, 19.22°, 19.86°, 21.14°, 21.57°, 22.29°, 23.10°, 23.38°, 23.57°, 25.71°, 26.49°, and 28.91°.

In some embodiments of the present disclosure, the X-ray powder diffraction (XRPD) pattern of the crystal form A of the compound of formula (I) is substantially as shown in FIG. 1.

In some embodiments of the present disclosure, in the X-ray powder diffraction (XRPD) pattern of the crystal form A of the compound of formula (I), the peak positions and relative intensities of the diffraction peaks are as shown in Table **1:**

**Table 1.** Peak positions and relative intensities of diffraction peaks in the XRPD pattern of the crystal form A of the compound of formula (I)

| No. | 20 (°) | Peak Intensity (cts) | *d*-Spacing (Å) | Relative Intensity (%) | No. | 20 (°) | Peak Intensity (cts) | *d*-Spacing (Å) | Relative Intensity (%) |
|---|---|---|---|---|---|---|---|---|---|
| 1 | 5.02 | 685.15 | 17.59 | 28.96 | 12 | 19.22 | 151.59 | 4.62 | 6.41 |
| 2 | 8.91 | 1049.63 | 9.92 | 44.37 | 13 | 19.86 | 273.77 | 4.47 | 11.57 |
| 3 | 9.80 | 293.03 | 9.02 | 12.39 | 14 | 21.14 | 2365.63 | 4.20 | 100.00 |
| 4 | 11.15 | 1375.99 | 7.93 | 58.17 | 15 | 21.57 | 364.66 | 4.12 | 15.41 |
| 5 | 12.72 | 227.97 | 6.96 | 9.64 | 16 | 22.29 | 206.10 | 3.99 | 8.71 |
| 6 | 13.87 | 420.54 | 6.39 | 17.78 | 17 | 23.10 | 432.77 | 3.85 | 18.29 |
| 7 | 14.70 | 311.88 | 6.03 | 13.18 | 18 | 23.38 | 869.29 | 3.80 | 36.75 |

(continued)

| No. | 2θ (°) | Peak Intensity (cts) | *d*-Spacing (Å) | Relative Intensity (%) | No. | 2θ (°) | Peak Intensity (cts) | *d*-Spacing (Å) | Relative Intensity (%) |
|---|---|---|---|---|---|---|---|---|---|
| 8 | 15.08 | 493.60 | 5.88 | 20.87 | 19 | 23.57 | 626.14 | 3.77 | 26.47 |
| 9 | 16.41 | 109.41 | 5.40 | 4.63 | 20 | 25.71 | 111.19 | 3.46 | 4.70 |
| 10 | 17.26 | 1024.83 | 5.14 | 43.32 | 21 | 26.49 | 276.16 | 3.36 | 11.67 |
| 11 | 18.38 | 612.25 | 4.83 | 25.88 | 22 | 28.91 | 186.82 | 3.09 | 7.90 |

In some embodiments of the present disclosure, the crystal form A of the compound of formula (I) has a differential scanning calorimetry (DSC) curve showing an endothermic peak with a peak temperature of 272.8°C±3°C and an exothermic peak with a peak temperature of 275.4°C±3°C.

In some embodiments of the present disclosure, the DSC pattern of the crystal form A of the compound of formula (I) is substantially as shown in FIG. 2.

In some embodiments of the present disclosure, the crystal form A of the compound of formula (I) has a thermogravimetric analysis (TGA) curve showing a weight loss of 1.00% at 150.0°C±3°C.

In some embodiments of the present disclosure, the TGA pattern of the crystal form A of the compound of formula (I) is substantially as shown in FIG. 3.

In some embodiments of the present disclosure, the crystal form A of the compound (I) of the present disclosure can be prepared by the following method:

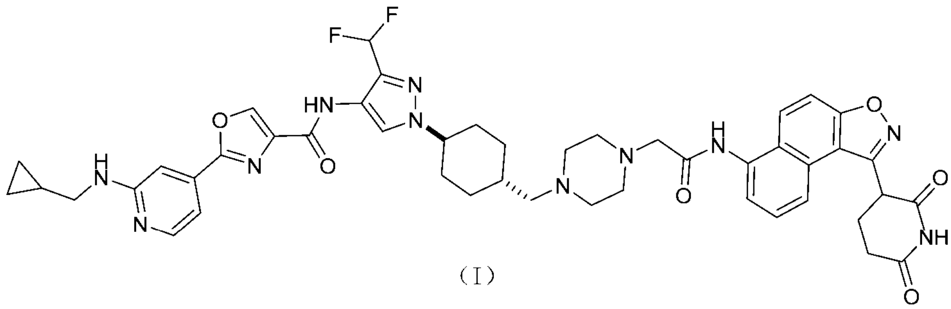

(I)

(a) adding the compound of formula (I) to a solvent;
(b) stirring at 15 to 30°C for 16 to 40 hours;
(c) filtering, and rinsing the filter cake with a solvent;
(d) collecting the filter cake, and drying under vacuum at 50 to 70°C for 10 to 200 hours;

wherein the solvent is ethanol.

The present disclosure also provides a use of the crystal form A of the compound of formula (I) in the manufacture of a medicament for treating diseases associated with interleukin-1 receptor-associated kinase 4, zinc finger transcription factor 1, or zinc finger transcription factor 3 protein degradation-targeting chimeras.

In some embodiments of the present disclosure, the diseases associated with interleukin-1 receptor-associated kinase 4, zinc finger transcription factor 1, and zinc finger transcription factor 3 protein degradation-targeting chimeras are diffuse large B-cell lymphoma.

In another aspect, the present disclosure provides a pharmaceutical composition comprising a therapeutically effective amount of the crystal form A of the compound of formula (I) of the present disclosure. The pharmaceutical composition of the present disclosure may or may not contain pharmaceutically acceptable excipients. Furthermore, the pharmaceutical composition of the present disclosure may further comprise one or more other therapeutic agents.

The present disclosure also provides a method for treating diffuse large B-cell lymphoma, comprising administering to a subject in need thereof a therapeutically effective amount of the crystal form A of the compound of formula (I) or the pharmaceutical composition of the present disclosure.

**Technical Effect**

The crystal form A of the compound of formula (I) of the present disclosure is simple to prepare, exhibits stable

properties and good hygroscopicity, and is less affected by light exposure and heat. The compound of the present disclosure shows excellent degradation effects on the target proteins IRAK4, IKZF1, and IKZF3, as well as excellent inhibition effects on cell proliferation in lymphoma cell lines OCI-LY10, TMD-8, and SU-DHL-2. The compound of formula (I) exhibits significant tumor inhibitory effects with dose dependency, along with good pharmacokinetic properties and oral absorption rates, making it suitable for use as a medicament.

**Definition and Description**

Unless otherwise specified, the following terms and phrases, when used herein, have the following meanings. A specific phrase or term should not be considered indefinite or unclear in the absence of a particular definition, but should be understood in the ordinary sense. When a trading name appears herein, it is intended to refer to its corresponding commodity or active ingredient thereof.

For any given crystal form, the relative intensities of diffraction peaks may vary due to factors such as preferred orientation caused by crystal morphology, which is well-known in the field of crystallography. Where preferred orientation effects exist, the peak intensities are altered, but the diffraction peak positions of the crystal form remain unchanged. Furthermore, for any given crystal form, slight deviations in peak positions may exist, which is also well-known in the field of crystallography. For example, due to temperature fluctuations during sample analysis, sample movement, or instrument calibration, peak positions may shift, with measurement errors for 2θ values sometimes around ±0.20°. Therefore, it is well-known to those skilled in the art that such variations should be taken into account when determining each crystal structure.

DSC measures the transition temperature at which a crystal absorbs or releases heat due to changes in its crystal structure or the melting of the crystal. For the same crystal form of the same compound, in consecutive analyses, the errors of the thermal transition temperature and melting point are typically within approximately 5°C or 3°C. When we state that a compound exhibits a given DSC peak or melting point, it refers to the DSC peak or melting point ±15°C or ±13°C. DSC provides an auxiliary method for distinguishing different crystal forms. Different crystal forms can be identified based on their distinct transition temperature characteristics. It should be noted that for mixtures, their DSC peaks or melting points may vary over a broader range. Furthermore, since decomposition accompanies the melting process of the substance, the melting temperature is related to the heating rate.

For the same crystal form, the TGA weight loss temperature may vary due to factors such as the measuring instrument, method/conditions, *etc.* For any specific crystal form, the weight loss temperature may have an error, which can be approximately ±15°C or approximately +3°C.

It should be noted that when preparing drug crystal forms, during the contact process between drug molecules and solvent molecules, it is difficult to avoid the situation where the solvent molecules and compound molecules form a co-crystal and remain in the solid material due to external conditions and internal factors, thereby forming solvates, including stoichiometric solvates and non-stoichiometric solvates. The solvates are all included within the scope of the present disclosure.

The term "pharmaceutically acceptable excipient" refers to inert substances administered together with the active ingredient to facilitate the administration of the active ingredient, including but not limited to any glidants, sweetening agents, diluents, preservatives, dyes/colorants, flavor enhancers, surfactants, wetting agents, dispersants, disintegrants, suspending agents, stabilizers, isotonic agents, solvents, or emulsifiers approved by the National Medical Products Administration for use in humans or animals (*e.g.*, livestock).

The term "targeting chimera" refers to a bifunctional molecule comprising two small molecule ligands, the first with high affinity for the target protein of interest, and the second for the recruitment of an E3 ligase, which ubiquitinates and targets the protein for proteolysis by the 26S proteasome.

The term "crystalline composition" refers to a mixture comprising the crystal form of Compound 1 of the present disclosure with other crystal forms or amorphous forms of the compound or other impurities. For example, the crystalline composition of the crystal form A of the compound of formula (I) includes, in addition to the crystal form A of the compound of formula (I), other crystal forms or amorphous forms of the compound of formula (I), or other impurities.

The term "pharmaceutical composition" refers to a mixture of one or more compounds of the present disclosure or salts thereof with pharmaceutically acceptable excipients. The purpose of the pharmaceutical composition is to facilitate the administration of the compounds of the present disclosure to an organism.

The therapeutic dosage of the compound of the present disclosure may be determined based on, for example, the specific use of the treatment, the mode of administration of the compound, the health and condition of the patient, and the judgment of the prescribing physician. The proportion or concentration of the compound of the present disclosure in the pharmaceutical composition may not be fixed and depends on a variety of factors, including dosage, chemical properties (*e.g.*, hydrophobicity), and route of administration.

The term "treatment" refers to the administration of the compound or formulation of the present disclosure to ameliorate or eliminate a disease or one or more symptoms associated with the disease, and includes:

(i) inhibiting a disease or disease state, *i.e.,* arresting its development;
(ii) alleviating a disease or disease state, *i.e.,* causing its regression.

The term "therapeutically effective amount" refers to an amount of the compound of the present disclosure for (i) treating a specific disease, condition, or disorder; (ii) alleviating, ameliorating, or eliminating one or more symptoms of a specific disease, condition, or disorder, or (iii) preventing or delaying the onset of one or more symptoms of a specific disease, condition, or disorder described herein. The amount of the compound of the present disclosure composing the "therapeutically effective amount" varies depending on the compound, the disease state and its severity, the mode of administration, and the age of the mammal to be treated, but can be routinely determined by those skilled in the art based on their knowledge and the present disclosure.

Unless the context requires otherwise, throughout the specification and claims thereafter, the term "comprise" and English variations thereof, such as "comprises" and "comprising", are to be construed in an open and inclusive sense, *i.e.,* "including, but not limited to".

Reference throughout the specification to "one embodiment" or "an embodiment" or "another embodiment" or "some embodiments" means that at least one embodiment includes specific reference elements, structures, or characteristics described in connection with the embodiment. Accordingly, the phrase "in one embodiment" or "in an embodiment" or "in another embodiment" or "in some embodiments" appearing in various places throughout the specification does not necessarily all refer to the same embodiment. In addition, the specific elements, structures, or characteristics may be combined in any suitable manner in one or more embodiments.

It should be understood that the singular forms "a", "an", and "the" used in the specification and appended claims of the present disclosure include plural referents unless the context clearly dictates otherwise. Thus, for example, reference to a reaction including "a catalyst" includes one catalyst, or two or more catalysts. It should also be understood that the term "or" is generally used in its sense including "and/or" unless the context clearly requires otherwise.

The intermediate compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred embodiments include, but are not limited to, the examples of the present disclosure.

The chemical reactions of the specific embodiments of the present disclosure are completed in a suitable solvent, and the solvent must be appropriate for the chemical changes of the present disclosure and the required reagents and materials thereof. In order to obtain the compounds of the present disclosure, it is sometimes necessary for those skilled in the art to modify or select the synthetic steps or reaction processes on the basis of the existing embodiments.

Unless otherwise specified, a wedge-shaped solid bond (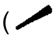) and a wedge-shaped dashed bond (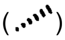) are used to indicate the absolute configuration of a stereocenter, while a straight solid bond (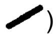) and a straight dashed bond (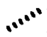) are used to indicate relative configuration. For example,

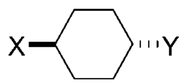

or

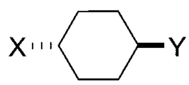

is used to represent a *trans*-1,4-disubstituted cyclohexane.

The present disclosure is described in detail by the examples below, but these examples do not imply any restrictions on the present disclosure.

All solvents used in the present disclosure are commercially available and can be used without further purification.

Compounds are named manually or using ChemDraw® software, while commercially available compounds adopt the supplier catalog names.

The following abbreviations are used in the present disclosure: RT represents room temperature; MeOH represents methanol; EtOH represents ethanol; IPA represents isopropanol; Acetone represents 2-propanone; MIBK represents methyl isobutyl ketone; EtOAc represents ethyl acetate; IPAc represents isopropyl acetate; MTBE represents methyl *tert*-butyl ether; THF represents tetrahydrofuran; 2-Me THF represents 2-methyltetrahydrofuran; DCM represents dichloromethane; $CHCl_3$ represents chloroform; Toluene represents methylbenzene; n-Heptane represents dipropylmethane; DMSO represents dimethyl sulfoxide; DMAc represents N,N-dimethylacetamide; NMP represents N-methylpyrrolidone; $H_2O$ represents water; 1,4-Dioxane represents 1,4-dioxacyclohexane; ACN represents acetonitrile.

**Instruments and Analytical Methods**

**1.1 X-ray powder diffraction (X-ray powder diffractometer, XRPD) method of the present** disclosure

Instrument model: PANalytacal X-ray Powder Diffractometer
Test method: Approximately 10 to 20 mg of the sample is used for XRPD detection.
The detailed XRPD parameters are as follows:
X-ray tube: Cu, kα, (λ = 1.540598 Å, λ = 1.544426 Å).
X-ray tube voltage: 45 kV, X-ray tube current: 40 mA
Divergence slit: 1/8°
Scan mode: continuous
Scanning range (°2Theta): 3-40
Scanning time per step (s): 46.7
Scanning step size (°2Theta): 0.0263 seconds
Test duration: approximately 5 min

**1.2 Differential scanning calorimetry (differential scanning calorimeter, DSC) method of the present disclosure**

Instrument model: TA 2500 Differential Scanning Calorimeter
Test method: A sample (approximately 1 mg) is placed in a DSC aluminum crucible for testing. Under a 50 mL/min $N_2$ atmosphere, the sample is heated from 30°C (room temperature) to 300°C at a heating rate of 10°C/min.

**1.3 Thermo gravimetric analysis (thermal gravimetric analyzer, TGA) method of the present disclosure**

Instrument model: TA 5500 Thermo gravimetric Analyzer
Test method: The sample (2 to 5 mg) is placed in a TGA platinum crucible for testing. Under a 25 mL/min $N_2$ atmosphere, the sample is heated from room temperature to 350°C at a heating rate of 10°C/min.

**1.4 Dynamic vapor sorption (DVS) method of the present disclosure**

Instrument model: DVS Intrinsic Plus Dynamic Vapor Sorption Analyzer from SMS (Surface Measurement Systems)
Test conditions: The sample (10 to 20 mg) is placed in a DVS sample pan for testing.
The detailed DVS parameters are as follows:
Temperature: 25°C
Equilibrium: dm/dt = 0.002 %/min (minimum: 10 min, maximum: 180 min)
Drying: 120 min at 0% RH
RH (%) test steps: 10%
RH (%) test step range: 0% - 95% - 0%

The hygroscopicity evaluation is classified as follows:

| **Classification of Hygroscopicity** | ΔW% |
|---|---|
| Deliquescence | Absorption of Sufficient Water to Form a Liquid |
| Highly Hygroscopic | ΔW% ≥ 15% |

(continued)

| | |
|---|---|
| Hygroscopic | 15% > ΔW% > 2% |
| Slightly Hygroscopic | 2% > ΔW% ≥ 0.2% |
| Non-Hygroscopic or Virtually Non-Hygroscopic | ΔW% < 0.2% |
| Note: AW% represents the hygroscopic weight gain of the test sample at 25±1°C and 80±2% RH. | |

BRIEF DESCRIPTION OF THE DRAWINGS

FIG. 1 shows the X-ray powder diffraction (XRPD) pattern of the crystal form A of the compound of formula (I) using Cu-Kα radiation;
FIG. 2 shows the differential scanning calorimetry (DSC) pattern of the crystal form A of the compound of formula (I);
FIG. 3 shows the thermogravimetric analysis (TGA) pattern of the crystal form A of the compound of formula (I);
FIG. 4 shows the dynamic vapor sorption (DVS) pattern of the crystal form A of the compound of formula (I).

DETAILED DESCRIPTION OF THE PREFERRED EMBODIMENT

The present disclosure is described in detail by the examples below, but it does not mean that there are any adverse restrictions on the present disclosure. The present disclosure has been described in detail herein, and its specific examples have also been disclosed; for those skilled in the art, it is obvious to make various modifications and improvements to the specific examples of the present disclosure without departing from the spirit and scope of the present disclosure.

**Example 1: Preparation of the compound of formula (I)**

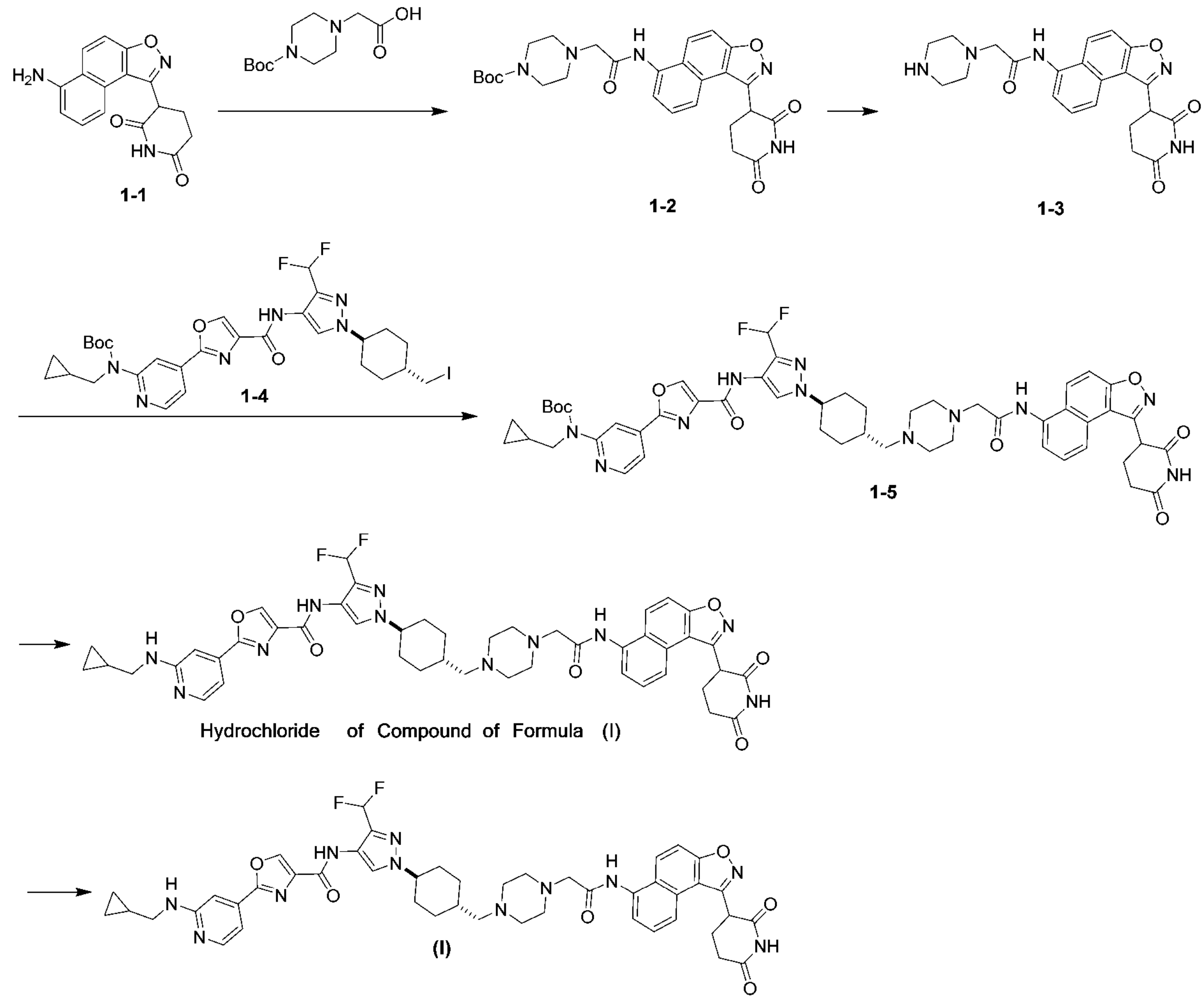

1-1 1-2 1-3 1-4 1-5

Hydrochloride of Compound of Formula (I)

(I)

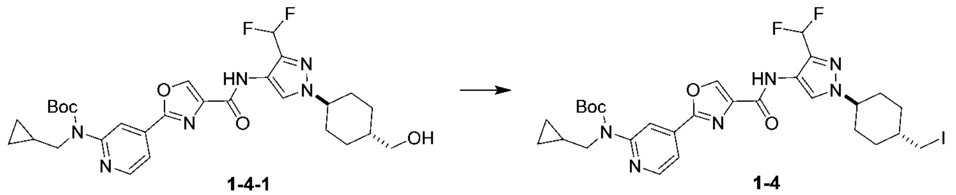

1-4-1 1-4

Step 1: Synthesis of compound 1-2

At room temperature under a nitrogen atmosphere, 2-(4-(tert-butoxycarbonyl)piperazin-1-yl)acetic acid (115.98 g, 474.75 mmol) was dissolved in N,N-dimethylformamide (1 L), followed by the addition of *N,N*-diisopropylethylamine (163.62 g, 1.27 mol) and 2-(7-azabenzotriazol-1-yl)-*N,N,N',N'*-tetramethyluronium hexafluorophosphate (180.51 g, 474.75 mmol). The reaction mixture was stirred at room temperature for 0.5 hours, and then the hydrochloride of compound 1-1 (105 g, 316.5 mmol) was added. The reaction mixture was stirred at room temperature for 12 hours. After the reaction was completed, the reaction mixture was poured into ice water (5 L) and extracted with ethyl acetate (4x1 L). The organic phases were combined, washed with saturated brine (3x1 L), dried over anhydrous sodium sulfate, filtered, and the filtrate was concentrated under reduced pressure to remove the solvent. The residue was dissolved in a mixed solvent of ethyl acetate and dichloromethane (volume ratio 1:1, 1.5 L), stirred at room temperature for 1 hour, during which solids gradually precipitated. The majority of dichloromethane was removed by concentration under reduced pressure, methyl *tert*-butyl ether (1.5 L) was added, and the mixture was stirred at room temperature for 12 hours, filtered, and the filter cake was collected. Ethyl acetate (1.5 L) was added to the filter cake, stirred at room temperature for 2 hours, filtered, and the filter cake was rinsed with ethyl acetate (200 mLx2). The filter cake was collected and dried under vacuum to obtain compound **1-2.** MS-ESI *m/z*: 522.3 $[M+H]^+$. $^1H$ NMR (400 MHz, DMSO_$d_6$) δ: 11.14 (s, 1H), 10.13 (s, 1H), 8.19 (d, *J*=9.6 Hz, 1H), 8.11 (d, *J*=8.0 Hz, 1H), 8.00 (d, *J*=9.26 Hz, 1H), 7.80-7.68 (m, 2H), 5.09 (dd, *J*=4.8 Hz, 11.2 Hz, 1H), 3.52-3.39 (m, 4H), 3.32 (s, 2H), 2.93-2.78 (m, 1H), 2.70-2.55 (m, 6H), 2.45-2.35 (m, 1H), 1.42 (s, 9H).

Step 2: Synthesis of the trifluoroacetate salt of compound **1-3**

At room temperature, compound **1-2** (153 g, 293.35 mmol) was dissolved in dichloromethane (1.2 L), and trifluoroacetic acid (462 g, 4.05 mol) was slowly added dropwise. The reaction mixture was stirred at room temperature for 12 hours. After the reaction was completed, the reaction mixture was concentrated under reduced pressure to remove the solvent to obtain the trifluoroacetate salt of compound **1-3.** MS-ESI *m*/*z*: 422.1 $[M+H]^+$.

Step 3: Synthesis of compound **1-4**

At room temperature under a nitrogen atmosphere, compound **1-4-1** (153 g, 260.81 mmol) was dissolved in dichloromethane (3 L), followed by the addition of triphenylphosphine (82.09 g, 312.97 mmol) and imidazole (26.63 g, 319.22 mmol). The reaction mixture was cooled to 0°C in an ice bath, and elemental iodine (86.06 g, 339.06 mmol) was added in batches. The reaction mixture was warmed to room temperature and stirred for 16 hours. After the reaction was completed, a saturated sodium sulfite solution (1 L) was slowly added to the reaction mixture, stirred for 20 minutes, and then the phases were separated. The aqueous phase was extracted with dichloromethane (3x3 L). The organic phases were combined, washed with saturated brine (2x2 L), dried over anhydrous sodium sulfate, filtered, and the filtrate was concentrated under reduced pressure to remove the solvent. Methyl *tert*-butyl ether (600 mL) was added to the residue, and the mixture was stirred at room temperature for 2 hours. The mixture was filtered, and the filter cake was rinsed with methyl *tert*-buty*l* ether (50 mLx2). The filtrate was collected. Anhydrous magnesium chloride (80 g) was added to the filtrate, and the mixture was stirred at room temperature for 8 hours. The mixture was filtered, and the filter cake was rinsed with methyl *tert*-butyl ether (50 mLx2). The filtrate was collected and concentrated under reduced pressure to obtain compound **1-4.** MS-ESI *m*/*z*: 697.0 $[M+H]^+$.

Step 4: Synthesis of compound **1-5**

At room temperature under a nitrogen atmosphere, the trifluoroacetate salt of compound **1-3** (129.35 g, 157.64 mmol) was dissolved in acetonitrile (1.3 L), followed by the addition of *N,N*-diisopropylethylamine (158.46 g, 1.23 mol) and compound **1-4** (122 g, 175.16 mmol). The reaction mixture was heated to 80°C and stirred for 12 hours. After the reaction was completed, part of the solvent was removed by concentration under reduced pressure, and ethyl acetate (500 mL) and water (500 mL) were added. The phases were separated, and the aqueous phase was extracted with ethyl acetate (500 mLx3). The organic phases were combined, washed with saturated brine (300 mL), dried over anhydrous sodium sulfate, filtered, and the filtrate was concentrated under reduced pressure to remove the solvent. The residue was dissolved in a mixed solvent of ethyl acetate and acetonitrile (volume ratio 9:1, 700 mL), stirred at room temperature for 2 hours, filtered, and the filter cake was rinsed with a mixed solvent of ethyl acetate and acetonitrile (volume ratio 9:1, 30 mLx3). The filter cake was collected and dried under vacuum to obtain compound **1-5.** MS-ESI *m*/*z*: 990.4 $[M+H]^+$.

Step 5: Synthesis of the hydrochloride of the compound of formula (I)

Compound **1-5** (80 g, 80.8 mmol) was dissolved in ethyl acetate (100 mL) at room temperature under a nitrogen atmosphere, then a solution of hydrogen chloride in ethyl acetate (4 M, 500 mL) was slowly added, and the reaction mixture was stirred at room temperature for 16 hours. After the reaction was completed, the mixture was filtered, and the filter cake was washed with ethyl acetate (50 mLx3). The filter cake was collected. Acetonitrile (350 mL) was added to the filter cake, and the mixture was stirred at room temperature for 2 hours. The mixture was filtered, and the filter cake was washed with acetonitrile (30 mLx3). The filter cake was collected and dried under vacuum to obtain the hydrochloride of the compound of formula (I). MS-ESI *m*/*z*: 890.2 $[M+H]^+$. $^1$H NMR (400 MHz, DMSO_$d_6$) δ: 11.59 (s, 1H), 11.14 (s, 1H), 11.05 (s, 1H), 9.97 (s, 1H), 9.59 (s, 1H), 9.15 (s, 1H), 8.45 (d, *J*=9.2 Hz, 1H), 8.23-8.12 (m, 2H), 8.06 (d, *J*=6.0 Hz, 1H), 7.99 (d, *J*=9.2 Hz, 1H), 7.81-7.73 (m, 2H), 7.70 (s, 1H), 7.29 (d, *J*=6.8 Hz, 1H), 7.22 (t, *J*=54.4 Hz, 1H), 5.11 (dd, *J*=4.0 Hz, 11.2 Hz, 1H), 4.42 (s, 2H), 4.31-4.20 (m, 1H), 3.81 (s, 6H), 3.47 (s, 2H), 3.39 (d, *J*=6.4 Hz, 2H), 3.11 (s, 2H), 2.94-2.78 (m, 1H), 2.72-2.54 (m, 2H), 2.45-2.35 (m, 1H), 2.17-2.02 (m, 5H), 2.01-1.90 (m, 1H), 1.88-1.74 (m, 2H), 1.29-1.09 (m, 2H), 0.61-0.50 (m, 2H), 0.39-0.30 (m, 2H).

Step 6: Synthesis of the compound of formula (I)

At room temperature under a nitrogen atmosphere, the hydrochloride of the compound of formula (I) (10 g) was dissolved in a mixed solvent of isopropanol (75 mL) and water (300 mL). The mixed solution was added dropwise to a solution of imidazole (3.43 g, 50.4 mmol) in water (300 mL), stirred at room temperature for 15 hours, and filtered. The filter cake was rinsed with water (50 mLx2) and isopropanol (50 mL), collected, and dried under vacuum to obtain the compound of formula (I) (free base).

**Example 2: Preparation of the crystal form A of the compound of formula (I)**

Method **1:** At room temperature, the compound of formula (I) (2.9 g) and acetonitrile (30 mL) were added to a reaction flask, stirred at room temperature for 19 hours, filtered, and the filter cake was rinsed with acetonitrile (2 mL). The filter cake was collected and purged with nitrogen for 10 to 48 hours to obtain the crystal form A of the compound of formula (I).

Method **2:** At room temperature, the compound of formula (I) (1 g) and dimethyl sulfoxide (4 mL) were added to a reaction flask, heated to 50°C, and after the compound was completely dissolved, ethanol (2.2 mL) was slowly added dropwise. After the dropwise addition was completed, the mixture was stirred for 30 minutes while maintaining the temperature, followed by another dropwise addition of ethanol (9.8 mL). After the dropwise addition was completed, the reaction mixture was stirred at 50°C for 30 minutes. The reaction mixture was then slowly cooled to room temperature, stirred at room temperature for 15 hours, filtered, and the filter cake was rinsed with ethanol (2 mLx2). The filter cake was collected and purged with nitrogen for 10 to 48 hours to obtain the crystal form A of the compound of formula (I).

Method **3:** At room temperature, the compound of formula (I) (0.5 g) and dimethyl sulfoxide (2 mL) were added to a reaction flask. The temperature was raised to 40°C until the compound was completely dissolved, followed by slow dropwise addition of ethyl acetate (20 mL). After the dropwise addition was completed, the mixture was stirred for 2 hours while maintaining the temperature, then slowly cooled to room temperature and stirred for 15 hours. The reaction mixture was filtered, and the filter cake was rinsed with ethyl acetate (1.5 mLx2). The filter cake was collected and purged with nitrogen for 10 to 48 hours to obtain the crystal form A of the compound of formula (I).

Method **4:** At room temperature, the compound of formula (I) (2.5 g) and ethanol (25 mL) were added to a reaction flask and stirred at room temperature for 20 hours. The reaction mixture was filtered, and the filter cake was rinsed with ethanol (10 mLx2). The filter cake was collected and purged with nitrogen for 10 to 48 hours to obtain the crystal form A of the compound of formula (I).

**Example 3: Hygroscopicity study of the crystal form A of the compound of formula (I)**

Experimental materials:

DVS Intrinsic Plus Dynamic Vapor Sorption Analyzer from SMS (Surface Measurement Systems).

Experimental methods:

10 to 20 mg of the crystal form A of the compound of formula (I) was placed in a DVS sample pan for testing.

Experimental results:

The DVS pattern of the crystal form A of the compound of formula (I) is substantially as shown in FIG. 4, with $\Delta W$ = 3.536%.

Experimental conclusion:

The crystal form A of the compound of formula (I) shows a hygroscopic weight gain of 3.536% at 25°C and 80% RH, demonstrating hygroscopicity, with no change in crystal form before and after DVS.

**Experimental Example 4: Stability test of the crystal form A of the compound of formula (I) in different solvents and mechanical stability evaluation**

**Anti-solvent addition:** Approximately 20 mg per portion of the crystal form A of the compound of formula (I) was weighed and added to 20 mL vials, then completely dissolved with 1.0 mL of the solvent listed in the table below (undissolved samples were filtered through a 0.45 μm PTFE filter to obtain a clear solution). While stirring (1000 rpm), the anti-solvent listed in the table below was added dropwise to the clear solution until solids precipitated, or until the total volume of anti-solvent reached 10 mL. Samples without precipitation were stirred in suspension at 5°C, while clear samples were transferred to -20°C for suspension stirring. If still clear, the samples were transferred to room temperature for volatilization. The precipitated solids were isolated and subjected to XRPD testing. The results are shown in Table **2.**

**Suspension stirring at room temperature:** Approximately 20 mg per portion of the crystal form A of the compound of formula (I) was weighed into HPLC vials, and 0.5 mL of the solvent listed in the table below was added to each vial. The resulting suspensions were magnetically stirred at room temperature (1000 rpm) for 3 days, then centrifuged to collect the solids, which were subjected to XRPD testing. The results are shown in Table **2.**

**Temperature cycling with stirring:** Approximately 20 mg per portion of the crystal form A of the compound of formula (I) was weighed into HPLC glass vials, and 0.5 mL of the solvent listed in the table below was added to each vial. The resulting suspensions were magnetically stirred (1000 rpm) under temperature cycling (heating the samples to 50°C, then cooling to 5°C at a rate of 0. 1°C/min, repeating this cycle, and finally maintaining the samples at 5°C). The solids were collected by centrifugation and subjected to XRPD testing. The results are shown in Table **2.**

**Slow cooling:** Approximately 20 mg of the crystal form A of the compound of formula (I) was weighed into an HPLC glass vial, and 0.5 mL of the solvent listed in the table below was added. The resulting suspension was magnetically stirred (1000 rpm) at 50°C to obtain a clear solution. If turbidity was observed, the suspension was filtered (0.45 μm PTFE filter membrane) to collect the filtrate. The obtained clear solution was cooled from 50°C to 5°C (0.1°C/min), and the sample was maintained at 5°C. The solid was collected and subjected to XRPD testing. The results are shown in Table **2.**

**Pressure stability:** 66.2 mg of the crystal form A of the compound of formula (I) was weighed and added to the mold of a manual hydraulic press. The sample in the mold was compressed under a pressure of 350 MPa. The solid was collected for XRPD characterization. The results are shown in Table **2.**

**Table 2.** Stability experiments of the crystal form A of the compound of formula (I) in different solvents and under tableting conditions

| **No.** | **Solvent** | **Anti-Solvent** | **XRPD Results** | |
|---|---|---|---|---|
| | | | **Starting Sample** | **Anti-Solvent Addition** |
| 1 | Dimethyl sulfoxide | Ethanol | Crystal Form A | Crystal Form A[1] |
| 2 | | Acetonitrile | | Crystal Form A[1] |
| 3 | | Isopropanol | | Crystal Form A[1] |
| 4 | | Acetone | | Crystal Form A[2] |
| 5 | | Methyl Isobutyl Ketone | | Crystal Form A[2] |
| 6 | | Ethyl Acetate | | Crystal Form A[2] |
| 7 | | Isopropyl Acetate | | Crystal Form A[2] |
| 8 | | Water | | Amorphous |
| 9 | N-Methylpyrrolidone | Methyl *tert*-Butyl Ether | | Crystal Form A[2] |
| 10 | | Water | | Amorphous |

| **No.** | **Solvent (Volume Ratio)** | **XRPD Results** | |
|---|---|---|---|
| | | **Starting Sample** | **Suspension Stirring at Room Temperature** |
| 1 | Acetone | Crystal Form A | Crystal Form A |
| 2 | Tetrahydrofuran | | |
| 3 | Tetrahydrofuran/Water (4:1) | | |
| 4 | Tetrahydrofuran/n-Heptane (1:1) | | |
| 5 | 1,4-Dioxane | | |
| 6 | 1,4-Dioxane/Water (4:1) | | |
| 7 | Ethanol/Water (97:3) | | |
| 8 | Ethanol/Water (93:7) | | |
| 9 | Water | | |
| 10 | Acetonitrile/Water (1:1) | | |
| 11 | Acetone/Water (1:1) | | |
| 12 | Methanol | | |

(continued)

| Starting Sample | Temperature Cycling with Stirring | | | |
|---|---|---|---|---|
| 1 | Ethanol | | Crystal Form A | Crystal Form A |
| 2 | Isopropanol | | | |
| 3 | N,N-Dimethylacetamide/Toluene (1:9) | | | |
| No. | Solvent (Volume Ratio) | | XRPD Results | |
| | | | Starting Sample | Slow Cooling |
| 1 | Tetrahydrofuran/Water (9:1) | | Crystal Form A | Crystal Form A[3] |
| No. | Pressure | | XRPD Results | |
| | | | Before Pressure | After Pressure |
| 1 | 350 MPa | | Crystal Form A | Crystal Form A |

[1]: The sample is clear after anti-solvent addition, and solids are precipitated after stirring overnight at room temperature.
[2]: The sample is clear at room temperature, transferred to 5°C and stirred for 3 days, then to -20°C and stirred for 2 days while remaining clear, and finally transferred to room temperature for open volatilization to precipitate solids.
[3]: The sample is stirred and left standing at 5°C for 3 days while remaining clear, then slowly volatilized at room temperature with the container left open, resulting in the precipitation of solids.

**Conclusion:** The crystal form A of the compound of formula (I) has good stability in solvents such as acetone, tetrahydrofuran, 1,4-dioxane, methanol, ethanol, isopropanol, water, as well as mixed solvents of tetrahydrofuran, 1,4-dioxane, ethanol, acetonitrile, acetone with water, and also exhibits good stability under a compression mold pressure of 350 MPa.

**Example 5: Solid stability test of the crystal form A of the compound of formula (I) under high temperature, high humidity, and strong light exposure conditions**

According to the "Guiding Principles for Stability Testing of Active Pharmaceutical Ingredients and Finished Dosage Forms" (General Chapter 9001 of the Chinese Pharmacopoeia 2020 Edition, Volume IV), the stability of the crystal form A of the compound of formula (I) under high temperature (60°C, open), high humidity (room temperature/relative humidity 92.5%, open), and strong light exposure (5000±500 Lux, 90 $\mu w/cm^2$, open) conditions was investigated.

1.5 g of the crystal form A of the compound of formula (I) was weighed and placed in an open weighing bottle, spread into a thin layer. Samples placed under high-temperature conditions were examined in an electric blast drying oven, while samples placed under high-humidity conditions were examined in a comprehensive drug stability test chamber. Sampling and testing were conducted on the 5th, 10th, and 30th days, and the test results were compared with the initial test results on day 0. Samples placed under strong light exposure conditions were left open, and sampling and testing were performed on the 5th and 10th days, with the test results compared to the initial test results on day 0. The test results are shown in Table 3 below.

**Table 3.** Solid stability test results of the crystal form A of the compound of formula (I) under high temperature, high humidity, and strong light exposure conditions

| Test Conditions | Sampling Time Point | Appearance | Content | Total Impurities | Crystal Form (XRPD) |
|---|---|---|---|---|---|
| - | Day 0 | Off-White Solid | 100.0% | 2.4% | Crystal Form A |

(continued)

| Test Conditions | Sampling Time Point | Appearance | Content | Total Impurities | Crystal Form (XRPD) |
|---|---|---|---|---|---|
| High Temperature (60°C, Open) | Day 5 | Off-White Solid | 102.8% | 2.5% | Crystal Form A |
| | Day 10 | Off-White Solid | 102.5% | 2.5% | Crystal Form A |
| High Humidity (Room Temperature/Relative Humidity: 92.5%, Open) | Day 5 | Off-White Solid | 99.7% | 2.4% | Crystal Form A |
| | Day 10 | Off-White Solid | 100.1% | 2.5% | Crystal Form A |
| Strong Light Exposure (5000±500 Lux, 90 $\mu w/cm^2$, Open) | Day 10 (Light Exposure Control) | Off-White Solid | 101.5% | 2.4% | Crystal Form A |
| | Day 10 | Off-White Solid | 102.3% | 2.6% | Crystal Form A |

**Conclusion:** The crystal form A of the compound of formula (I) has good stability under high temperature, high humidity, and strong light exposure conditions.

**Example 6: Solid stability test of the crystal form A of the compound of formula (I) under accelerated conditions**

According to the "Guiding Principles for Stability Testing of Active Pharmaceutical Ingredients and Finished Dosage Forms" (General Chapter 9001 of the Chinese Pharmacopoeia 2020 Edition), the stability of the crystal form A of the compound of formula (I) was investigated under accelerated conditions of high temperature and high humidity (40°C/75% relative humidity, sealed + desiccant).

Approximately 1.5 g of the crystal form A of the compound of formula (I) was weighed, placed into a double-layer low-density polyethylene bag, with each layer of the low-density polyethylene bag separately sealed with a zip tie, and then placed into an aluminum foil bag. Samples were taken for testing at the 1st, 2nd, and 3rd months, and the test results were compared with the initial test results at day 0. This experiment was repeated twice, each time using a different batch of the crystal form A of the compound of formula (I). The test results are shown in Table 4 below.

**Table 4.** Solid stability test results of the crystal form A of the compound of formula (I) under accelerated conditions (40°C/75% relative humidity, sealed + desiccant)

| Test Conditions | Sampling Time Point | Appearance | Content | Total Impurities | Crystal Form (XRPD) |
|---|---|---|---|---|---|
| - | Day 0 | Off-White Solid | 100.0% | 2.4% | Crystal Form A |
| 40°C/75% Relative Humidity, Sealed + Desiccant | 1 month | Off-White Solid | 99.3% | 2.7% | Crystal Form A |
| | 2 month | Off-White Solid | 99.5% | 2.7% | Crystal Form A |
| | 3 month | Off-White Solid | 99.1% | 2.7% | Crystal Form A |

**Conclusion:** The crystal form A of the compound of formula (I) has good stability under accelerated conditions of 40°C/75% relative humidity.

**Experimental Example 7: Solid stability test of the crystal form A of the compound of formula (I) under long-term conditions**

According to the "Guiding Principles for Stability Testing of Active Pharmaceutical Ingredients and Finished Dosage Forms" (General Chapter 9001 of the Chinese Pharmacopoeia 2020 Edition), the stability of the crystal form A of the compound of formula (I) was investigated under long-term conditions (25°C/60% relative humidity, sealed + desiccant).

Approximately 1.5 g of the crystal form A of the compound of formula (I) was weighed, placed into a double-layer low-density polyethylene bag, with each layer of the low-density polyethylene bag separately sealed with a zip tie, and then

placed into an aluminum foil bag. Samples were taken for testing at the 1st and 2nd months, and the test results were compared with the initial test results at day 0. This experiment was repeated twice, each time using a different batch of the crystal form A of the compound of formula (I). The experimental results are shown in Table **5** below.

**Table 5.** Solid stability test results of the crystal form A of the compound of formula (I) under long-term conditions (25°C/60% relative humidity, sealed + desiccant)

| **Test Conditions** | **Sampling Time Point** | **Appearance** | **Content** | **Total Impurities** | **Crystal Form (XRPD)** |
|---|---|---|---|---|---|
| - | Day 0 | Off-White Solid | 100.0% | 2.4% | Crystal Form A |
| 25°C/60% Relative Humidity, Sealed + | 1 month | Off-White Solid | 99.5% | 2.8% | Crystal Form A |
| Desiccant | 2 month | Off-White Solid | 99.2% | 2.9% | Crystal Form A |

**Conclusion:** The crystal form A of the compound of formula (I) has good stability under long-term conditions of 25°C/60% relative humidity.

**Bioassay**

**Experimental Example 1: Evaluation of the target protein degradation effect of the compound of formula (I) in K562 IRAK4-HiBiT cells**

**Objective:** In this experiment, the degradation effect of the compound of formula (I) on the target protein IRAK4 in K562 IRAK4-HiBiT cells was detected.

**Experimental materials:**

1. Cells and culture medium

Cells: K562 IRAK4-HiBiT cells
Culture medium: RPMI 1640 + 10% fetal bovine serum + 2 mM glutamine + 1 mM sodium pyruvate + penicillin/-streptomycin
Positive control: 1000 nM; negative control: 0.1% DMSO

Table **6.** Reagents and consumables

| Reagents and Consumables | Manufacturer | Cat. No. |
|---|---|---|
| RPMI 1640 | Gibco | Cat# 31800 |
| Fetal Bovine Serum (FBS) | Biosera | #FB-1058/500 |
| Penicillin/Streptomycin | Biosera | #LM-A4118 |
| DPBS | Invitrogen | #14190 |
| 0.25% Trypsin/EDTA Solution | Invitrogen | #25200 |
| Glutamine | Invitrogen | Cat# 35050061 |
| Sodium Pyruvate | Invitrogen | Cat# 11360070 |
| Nano-Glo® HiBiT Assay Kit | Promega | Promega# N3040 |
| 384-Well White Plate, Flat Bottom | Corning | Cat# 3570 |
| 384_LDV Compound Plate | Labcyte | Cat# LP-0200 |

Table 7. Instruments

| Instrument Name | Manufacturer | Instrument Model |
|---|---|---|
| ECHO Pipette | Labcyte | Echo 550 |
| Bravo Automated Liquid Handling Platform | Agilent | 16050-101 |
| Envision Plate Reader | PerkinElmer | 2104 |
| Autosampler | Thermo Fisher | Multidrop Combi |
| Cell Counter | Thermo | Countess II FL |

**Experimental scheme:**

**Day 1**

1. Compound preparation

(1) The test compound in powder form was dissolved in DMSO to 10 mM as a storage concentration, and 9 μL of the 10 mM test compound was manually pipetted to column 1 and column 13 of an LDV plate using a pipette.
(2) 6 μL of DMSO was added to columns 2 to 12 and 14 to 24 using Multidrop Combi.
(3) The test compound was 3-fold diluted (3 μL + 6 μL) from columns 1 to 11 and 13 to 23 using Bravo.
(4) 25 nL of compound solution (columns 1 to 24 of the LDV plate) was transferred to an assay plate using Echo according to the plate layout.
(5) 25 nL of 1 mM positive control solution was transferred to the assay plate as a 100% degradation control *(i.e.,* LC, HPE), and 25 nL of DMSO was transferred to the assay plate as a 0% control *(i.e.,* HC, ZPE) using Echo.

2. Cell seeding

(1) The cell culture medium was discarded, and the cells were washed once with DPBS, digested with trypsin, and counted to prepare a cell suspension of $2\times10^{-5}$ cells/mL.
(2) 25 μL/well of cell suspension was added to the assay plate containing the test compound using Multidrop Combi at medium speed.
(3) The assay plate containing cells was returned to an incubator with 5% $CO_2$ at 37°C and incubated for 16 to 18 hours.

**Day 2**

(1) 25 μL/well of assay reagent (NanoGlo lysis buffer + substrate + LgBit protein) was added to the assay plate using Multidrop Combi at high speed, and the plate was shaken for 10 minutes.
(2) The plate was centrifuged at 2000 rpm for 1 minute to remove air bubbles.
(3) The plate was read on Envision using US Luminescence detection method.

3. Data analysis

The degradation rate (DR) of the test compound was calculated using the following formula: DR (%) = (RLU vehicle control - RLU compound)/(RLU vehicle control - RLU positive control)*100%, where the vehicle control served as the blank control. The degradation rates of compounds at different concentrations were calculated in Excel, and XLFit software was used to generate inhibition curves and calculate relevant parameters, including minimum degradation rate, maximum degradation rate, and $DC_{50}$.
The test results are shown in Table **8.**

Table **8.** Target protein degradation effect of the compound of the present disclosure in K562 IRAK4-HiBiT cells

| Compound No. | $DC_{50}$ (nM) | Maximum Degradation Rate (%) |
|---|---|---|
| Hydrochloride of Compound of Formula (I) | 4.87 | 108.46 |

**Conclusion:** The compound of the present disclosure exhibits an excellent target protein degradation effect in K562 IRAK4-HiBiT cells.

**Experimental Example 2: In cell western analysis of IKZF1 and IKZF3 protein expression levels of the compound of formula (I) in MM.1S Cells**

**Objective:** The experiment was conducted to evaluate the degradation effects of the compound of formula (I) on IKZF1 and IKZF3 proteins in MM.1S cells by detecting their impact on the expression levels of IKZF1 and IKZF3 proteins in MM.1S cells.

**Experimental materials:**

Cell line: MM.1S cell (obtained from ATCC; Cat. No. CRL-2974)
Negative control: 0.1% DMSO

Table **9.** Reagents and consumables

| Reagents and Consumables | Supplier | Cat. No. |
|---|---|---|
| 96-Well Black Clear-Bottom Plate for ELISA | JingAn Biological | J09603 |
| D-PBS Buffer | Bioleaper | P220610030051 |
| Tissue Fixative Solution | Meilumbio | MA0192-Jan-28G |
| TritonX-100 Solution (10% Sterile) | Beyotime | ST797-500ML |
| Intercept Blocking Buffer | LI-COR | 211114 |
| Ikaros (D6N9Y) Rabbit mAb | Cell Signaling | 14859S |
| Aiolos (D1C1E) Rabbit mAb | Cell Signaling | 15103S |
| GAPDH Mouse mAb | Proteintech | 60004-1-Ig |
| Tween 20 | Sinopharm Chemical Reagent Co., Ltd. | 30189328 |
| IRDye 800CW Goat Anti-Mouse | LI-COR | D00812-08 |
| IRDye 680RD Goat Anti-Rabbit | LI-COR | D00891-05 |

Table **10.** Instruments

| Instrument Name | Supplier | Instrument Model |
|---|---|---|
| Dual-Color Infrared Laser Imager | LI-COR | Odyssey-CLx |
| Decolorization Shaker | QILNBEIER | TS-1000 |
| Refrigerator | Haier Group | BCD-256KT |

**Experimental scheme:**

1) MM.1S cells in the logarithmic growth phase were seeded into a 96-well plate at $1.2 \times 10^5$ cells per well and cultured overnight.

2) On the second day, drug treatment was initiated with a starting concentration of 300 nM, followed by a 3-fold dilution in triplicate wells, with 10 concentration gradients (containing DMSO), and incubated for 24 hours in a culture incubator.
3) The mixture was centrifuged, and the cell supernatant was carefully removed. Then, 150 μL of 4% paraformaldehyde fixative solution was added along the well wall without touching the bottom cells, followed by incubation at room temperature for 20 min.
4) Permeabilization solution was prepared by adding 0.5 mL of 10% Triton X-100 to 49.5 mL of PBS and mixing thoroughly.
5) 200 μL of the permeabilization solution was added along the well wall without touching the bottom cells, and incubated at room temperature on a shaker for 5 min.
6) The washing step was repeated 4 times.
7) 150 μL of Licor INERCEPT blocking buffer (intertent Blacking buffer) was added along the well wall without touching the bottom cells, and incubated at room temperature on a shaker for 1.5 hours.
8) Ikaros (D6N9Y) Rabbit mAb, Aiolos (D1C1E) Rabbit mAb, and GAPDH Mouse mAb (Proteintech, 60004-1-Ig) were diluted at a ratio of 1:100 using antibody diluent.
9) 50 μLof the mixed antibodies was added to each well in triplicate, and incubated overnight at 4°C on a shaker.
10) PBST (PBS containing 0.1% Tween 20) was prepared.
11) The primary antibody was removed, and 200 μL of PBST was added along the well wall without touching the bottom cells. The mixture was incubated at room temperature on a shaker for 5 min.
12) The washing step was repeated 4 times.
13) The secondary antibody diluent was prepared by adding Tween 20 to the Licor INTERCEPT blocking buffer at a final concentration of 0.2%.
14) The fluorescent secondary antibody was diluted in the dark (1:800 dilution). To 400 μL of the secondary antibody diluent, 0.5 μLeach of IRDye 800CW and IRDye 680CW was added (the corresponding fluorescent secondary antibody for the primary antibody).
15) 50 μL of the diluted fluorescent secondary antibody was added to each well, followed by incubation at room temperature on a shaker in the dark for 60 min.
16) The secondary antibody was removed, and 200 μL of PBST was added along the well wall without touching the bottom cells. The plate was then incubated at room temperature on a shaker in the dark for 5 min.
17) The washing step was repeated 4 times. Immediately after washing, the plate was scanned using the Odyssey Gel Imaging System at dual wavelengths of 700 nm and 800 nm.

3. Data analysis

GraphPad Prism 6 software was utilized to fit the curve with the inhibition rate data, and the $DC_{50}$ value was calculated.

$$\text{Protein inhibition rate} = (1\text{-RLs/RLv})*100\%$$

$$\text{RR (Raw Ratio)} = 700\ \text{nm}/800\ \text{nm}$$

RLs = RR of sample-treated cells
RLv = RR of vehicle-treated cells

The test results are shown in Table **11.**

Table **11.** Degradation effects of the compound of formula (I) of the present disclosure on IKZF1 and IKZF3 proteins in MM.1S cells

| Compound No. | Protein | $DC_{50}$ (nM) | Maximum Degradation Rate Dmax (%) |
|---|---|---|---|
| Hydrochloride of Compound of Formula (I) | IKZF 1 | 23.40 | 72.32 |
| | IKZF3 | 20.64 | 73.46 |

**Conclusion:** The compound of the present disclosure exhibits excellent target protein degradation effects on IKZF1 and IKZF3 proteins in MM.1S cells.

**Experimental Example 3: Evaluation of the anti-proliferative effect of the compound of formula (I) in lymphoma cell lines OCI-LY10 and TMD-8**

**Objective:** This experiment was conducted to detect the inhibitory effects of the compound of formula (I) on cell proliferation in diffuse large B-cell lymphoma cell lines **OCI-LY10 and TMD-8.**

**Experimental materials:**

Table **12.** Cell lines and culture methods

| Cell Lines | Tumor Type | Growth Characteristics | Culture Media |
|---|---|---|---|
| OCI-LY10 | Lymphoma | Suspension | IMDM + 20% FBS + 55 μM β-Mercaptoethanol |
| TMD-8 | Lymphoma | Suspension | MEM + 10% FBS |

Table **13.** Culture media and reagents

| Culture Media and Reagents | Manufacturer | Cat. No. |
|---|---|---|
| Dulbecco's PBS | Hyclone | SH30256.01 |
| Fetal Bovine Serum (FBS) | GIBCO | 10099-141 |
| Antibiotic-Antimycotic | GIBCO | 15240-062 |
| 0.25% Trypsin | GIBCO | 25200072 |
| DMSO | SIGMA | D2650 |
| β-Mercaptoethanol | SIGMA | 60-24-2 |
| MEM | GIBCO | 11095-080 |
| IMDM | GIBCO | 12440-053 |

1. Multi-well plate

Greiner CELLSTAR® 96-well plate, flat-bottom white (with lid and clear bottom), # 3610.

2. Reagents and instruments for cell viability assay

(1) Promega CellTiter-Glo Luminescent Cell Viability Assay Kit (Promega-G7573).
(2) 2104 EnVision® Plate Reader, PerkinElmer.

**Experimental scheme:**

1. Cell culture

The tumor cell lines were cultured under the above culture conditions in an incubator at 37°C and 5% $CO_2$. The cells were passaged periodically, and those in the logarithmic growth phase were harvested for seeding.

2. Cell seeding

(1) The cells were stained with trypan blue, and the viable cells were counted.
(2) The cell concentration was adjusted to an appropriate concentration.

Table **14.** Cell lines and seeding densities

| Cell Lines | Seeding Densities (cells/well) |
|---|---|
| OCI-LY 10 | 5000 |
| TMD-8 | 5000 |

(3) 100 μL of cell suspension was added to each well of the culture plate as shown in the figure above.
(4) The culture plate was incubated overnight in an incubator at 37°C, 5% $CO_2$, and 100% relative humidity.

3. Preparation of compound storage plate

Preparation of stock solution storage plate with 1000 times the initial concentration of the compound: The compound was serially diluted with DMSO from the highest concentration to the lowest concentration. The solution was prepared as needed each time.

4. Preparation of 1000× compound working solution and compound treatment of cells

(1) Preparation of 5× working solution from the initial compound concentration: When performing a 3-fold dilution of the compound, 30 μL of the compound was pipetted from the 1000× stock solution. Subsequently, 20 μLof DMSO was added to the subsequent wells, and 10 μLwas sequentially pipetted from the previous concentration to the next. When performing a 5-fold dilution of the compound, 30 μLof the compound was pipetted from the 1000× stock solution, followed by the addition of 24 μLof DMSO to the subsequent wells. Then, 6 μL was sequentially pipetted from the previous concentration to the next. In the vehicle control, 20 μL of DMSO was added. The 1000× compound was diluted 200-fold with culture medium, specifically by adding 1 μL of the 1000× diluted compound to 199 μL of culture medium, followed by pipetting up and down to mix thoroughly.
(2) Drug addition: 25 μLof the 5× compound was added to the cell culture plate.
(3) The 96-well cell plate was returned to the incubator for culturing OCI-LY10 (3-fold or 5-fold dilution, incubated with the drug for 5 days) and TMD-8 (3-fold or 5-fold dilution, incubated with the drug for 5 days).

5. CellTiter-Glo luminescent cell viability assay

The following steps were conducted in accordance with the instructions of the Promega CellTiter-Glo Luminescent Cell Viability Assay Kit (Promega-G7573).

(1) CellTiter-Glo buffer was thawed and brought to room temperature.
(2) CellTiter-Glo substrate was brought to room temperature.
(3) CellTiter-Glo working solution was prepared by adding 10 mL of CellTiter-Glo buffer to a vial of CellTiter-Glo substrate to dissolve the substrate.
(4) The mixture was slowly vortexed and shaken to be fully dissolved.
(5) The cell culture plate was taken out and left for 30 minutes to equilibrate to room temperature.
(6) 60 μL of CellTiter-Glo working solution was added per well (equal to half the volume of cell culture medium per well). The cell plate was wrapped in aluminum foil to be protected from light.
(7) The culture plate was shaken on an orbital shaker for 2 minutes to induce cell lysis.
(8) The culture plate was left at room temperature for 10 minutes to stabilize the luminescent signal.
(9) The luminescent signal was detected on a 2104 EnVision plate reader.

6. Data analysis

The inhibition rate (IR) of the test compound was calculated using the following formula: IR (%) = (1 - RLU compound/RLU vehicle control)* 100%. The inhibition rates of compounds at different concentrations were calculated in Excel, and then GraphPad Prism software was used to plot inhibition curves and calculate relevant parameters, including minimum inhibition rate, maximum inhibition rate, and $IC_{50}$.
The test results are shown in Table **15.**

Table **15.** Inhibitory effects of the compound of formula (I) of the present disclosure on cell proliferation in cell lines OCI-LY 10 and TMD-8

| Compound No. | OCI-LY10 | | TMD-8 | |
|---|---|---|---|---|
| | $IC_{50}$ (nM) | Maximum Inhibition Rate (%) | $IC_{50}$ (nM) | Maximum Inhibition Rate (%) |
| Hydrochloride of Compound of Formula (I) | 13.66 | 94.54 | 10.34 | 89.86 |

"/" indicates not detected.

**Conclusion:** The compound of the present disclosure exhibits excellent inhibitory effects on cell proliferation in both lymphoma cell lines OCI-LY 10 and TMD-8.

**Experimental Example 4: Evaluation of the anti-proliferative effect of the compound of formula (I) in lymphoma cell line SU-DHL-2**

**Objective:** This experiment was conducted to detect the inhibitory effect of the compound of formula (I) on cell proliferation in the lymphoma cell line **SU-DHL-2.**

Table **16.** Cell line and culture method

| Cell Line | Tumor Type | Growth Characteristic | Culture Method |
|---|---|---|---|
| SU-DHL-2 | Lymphoma | Suspension | RPMI1640 + 10% FBS (EXCELL) + 1% Penicillin/Streptomycin |

Table **17.** Culture media and reagents

| Culture Media and Reagents | Manufacturer | Cat. No. |
|---|---|---|
| RPMI 1640 | GIBCO | 22400-089 |
| Dulbecco's PBS | Thermo | SH30028.02B |
| Fetal Bovine Serum (FBS) | Hyclone | 11H233 |
| Antibiotic-Antimycotic | GIBCO | 15240-062 |
| DMSO | SIGMA | D2650 |

1. Multi-well plate

Greiner CELLSTAR 384-well plate, flat-bottom black (with lid), # 781090.

2. Reagents and instruments for cell viability assay

(1) Promega CellTiter-Glo Luminescent Cell Viability Assay Kit (Promega-G7573).
(2) 2104 EnVision® Plate Reader, PerkinElmer.

**Experimental scheme:**

1. Cell culture

The tumor cell lines were cultured under the above culture conditions in an incubator at 37°C and 5% $CO_2$. The cells were passaged periodically, and those in the logarithmic growth phase were harvested for seeding.

2. Cell seeding

(1) The cells were stained with trypan blue, and the viable cells were counted.
(2) The cell concentration was adjusted to an appropriate concentration.

Table **18.** Cell line and seeding density

| Cell Line | Seeding Density (cells/well) |
|---|---|
| SU-DHL-2 | 1500 |

(3) 50 μL of cell suspension was added to each well of the culture plate as shown in the figure above.
(4) The culture plate was incubated overnight in an incubator at 37°C, 5% $CO_2$, and 100% relative humidity.

3. Preparation of compound storage plate

The drug was administered with an Echo655 instrument. A dosing volume of 50 nL was used, with a final DMSO concentration of 0.1%. The culture plate was centrifuged at 1000 rpm for 1 min and incubated at 37°C, 5% $CO_2$, and 100% relative humidity for 4 days.

4. CellTiter-Glo luminescent cell viability assay

The following steps were conducted in accordance with the instructions of the Promega CellTiter-Glo Luminescent Cell Viability Assay Kit (Promega-G7573).

(1) CellTiter-Glo buffer was thawed and brought to room temperature.
(2) CellTiter-Glo substrate was brought to room temperature.
(3) CellTiter-Glo working solution was prepared by adding CellTiter-Glo buffer to a vial of CellTiter-Glo substrate to dissolve the substrate.
(4) The mixture was slowly vortexed and shaken to be fully dissolved.
(5) The cell culture plate was taken out and left for 30 minutes to equilibrate to room temperature.
(6) 25 μL (equal to half the volume of cell culture medium in each well) of CellTiter-Glo working solution was added to each well. The cell plate was wrapped in aluminum foil to be protected from light.
(7) The culture plate was shaken on an orbital shaker for 2 minutes to induce cell lysis.
(8) The culture plate was left at room temperature for 10 minutes to stabilize the luminescent signal.
(9) The luminescent signal was detected using a 2104 EnVision Plate Reader.

5. Data analysis

The inhibition rate (IR) of the test compounds was calculated using the following formula: IR (%) = (1 - (RLU compound - RLU blank control)/(RLU vehicle control - RLU blank control))* 100%. The inhibition rates of compounds at different concentrations were calculated in Excel, and then GraphPad Prism software was used to generate inhibition curves and calculate relevant parameters, including the minimum inhibition rate, maximum inhibition rate, and $IC_{50}$.

Table **19.** Inhibitory effect of the compound of the present disclosure on cell proliferation in cell line SU-DHL-2

| Compound No. | SU-DHL-2 $IC_{50}$ (nM) | Maximum Inhibition Rate (%) |
|---|---|---|
| Hydrochloride of Compound of Formula (I) | 28.70 | 93.55 |

**Conclusion:** The hydrochloride of the compound of formula (I) of the present disclosure exhibits an excellent inhibitory effect on cell proliferation in the lymphoma cell line SU-DHL-2.

**Experimental Example 5: Pharmacokinetic evaluation of the compound of formula (I) in mice**

**Objective:**

C57BL/6 or C57 male mice were selected as the test animals for this study. The LC/MS/MS method was employed to quantitatively determine the plasma drug concentrations at various time points after intravenous injection or intragastric administration of the test compound, aiming to evaluate the pharmacokinetic characteristics of the compound of the present disclosure in mice.

**Experimental materials:**

C57BL/6 or C57 mice (male, 20 to 30g, 7 to 10 weeks old, Beijing Vital River).

**Experimental operation:**

A clear solution of the test compound was injected intravenously into the tail vein of C57BL/6 mice (fasted overnight) (vehicle: 10% DMSO/10% solutol/80% $H_2O$), or administered intragastrically to C57 mice (fed). For intravenous injection, 50 μL of blood was collected via the cheek puncture at 0 h (pre-administration) and 0.083 h, 0.25 h, 0.5 h, 1 h, 2 h, 4 h, 6 h, 8 h, and 24 h after administration, and placed in an anticoagulant tube added with heparin sodium. The mixture was thoroughly vortexed and mixed, and centrifuged at 6000 g for 3 minutes at 2 to 8°C. For intragastric administration, blood was collected via the cheek puncture at 0 h (pre-administration) and 0.083 h, 0.25 h, 0.5 h, 1 h, 2 h, 4 h, 6 h, 8 h, and 24 h after administration, and placed in an anticoagulant tube added with heparin sodium. The mixture was thoroughly vortexed and mixed, and centrifuged at 6000 g for 3 minutes at 2 to 8°C. Plasma drug concentrations were determined by the LC-MS/MS method, and relevant pharmacokinetic parameters were calculated using Phoenix WinNonlin 8.2.0 pharmacokinetic software with non-compartmental model linear-log trapezoidal method.

Table **20.** Pharmacokinetic parameters of the compound of the present disclosure in mice

| Compound No. | Pharmacokinetic Parameters in Mice | | | | | | | |
|---|---|---|---|---|---|---|---|---|
| | Intravenous Injection (2 mg/kg) (fasted) | | | | Intragastric administration (10 mg/kg) (fed) | | | |
| | Plasma Clearance (mL/min/ kg) | Elimination Half-Life (h) | Apparent Volume of Distribution (L/kg) | Area Under the Drug-Time Curve (0-inf, μM·h) | Peak Concentration (μM) | Time to Peak Concentration (h) | Area Under the Drug-Time Curve (0-inf, μM·h) | Bioavailability F (%) |
| Hydrochloride of Compound of Formula (I) | 7.0 | 4.22 | 1.6 | 5.40 | 0.54 | 1.00 | 4.47 | 18.90 |

**Conclusion:** The compound of the present disclosure has high oral systemic plasma exposure ($AUC_{0-inf}$). It has superior pharmacokinetic properties in rodents such as mice.

**Experimental Example 7: Pharmacokinetic evaluation in beagle dogs**

**Objective:**

Male beagle dogs were selected as the experimental animals in this study. The LC/MS/MS method was employed to quantitatively determine the plasma drug concentrations at different time points after intravenous injection or intragastric administration of the test compound, aiming to evaluate the pharmacokinetic characteristics of the compound of the present disclosure in beagle dogs.

**Experimental materials:**

Beagle dogs (male, 7 to 10 kg, Beijing Mars Biotechnology Co., Ltd.).

**Experimental operation:**

A solution of the test compound was slowly injected intravenously into beagle dogs (fed) via the peripheral vein (vehicle: 5% DMSO/10% Solutol/85% $H_2O$), or administered intragastrically to beagle dogs (fed). For intravenous administration, 0.5 mL of blood was collected from the peripheral vein at 0.083, 0.25, 0.5, 1, 2, 4, 6, 8, and 24 h after administration, placed in EDTA-2K anticoagulant tubes, and centrifuged at 3200 g for 10 minutes at 2 to 8°C to separate the supernatant. For intragastric administration, 0.5 mL of blood was collected from the peripheral vein at 0.083, 0.25, 0.5, 1, 2, 4, 6, 8, and 24 h after administration, placed in EDTA-2K anticoagulant tubes, and centrifuged at 3200 g for 10 minutes at 2 to 8°C to separate the supernatant. Plasma drug concentrations were determined by the LC-MS/MS method, and relevant pharmacokinetic parameters were calculated using Phoenix WinNonlin 6.3 or 8.3.5 pharmacokinetic software with non-compartmental model linear-log trapezoidal method.

The test results are shown in Table **21.**

Table **21.** Pharmacokinetic parameters of the compound of the present disclosure in Beagle dogs

| Compound No. | Pharmacokinetic Parameters | | | | | | | |
|---|---|---|---|---|---|---|---|---|
| | Intravenous Injection (0.5 mg/kg) | | | | Intragastric Administration | | | |
| | Plasma Clearance (mL/min /kg) | Elimination Half-Life (h) | Apparent Distribution Volume (L/kg) | Area Under the Drug-Time Curve (0-inf, μM·h) | Peak Concentration (μM) | Time to Peak Concentration (h) | Area Under the Drug-Time Curve (0-inf, μM·h) | Bioavailability F (%) |
| Hydrochloride of Compound of Formula (I) (5 mg/kg) | 21.3 | 5.03 | 5.83 | 0.44 | 0.41 | 4.00 | 3.20 | 72.6 |
| Hydrochloride of Compound of Formula (I) (15 mg/kg) | | | | | 0.87 | 4.00 | 8.02 | 60.8 |
| Crystal Form A of Compound of Formula (I) (5 mg/kg) | | | | | 0.70 | 5.00 | 8.18 | 62.0 |

**Conclusion:** The compound of the present disclosure has high oral systemic plasma exposure ($AUC_{0-inf}$). It has superior pharmacokinetic properties in non-rodent animals such as Beagle dogs.

**Experimental Example 8: *In vivo* pharmacodynamic study in SCID mouse xenograft tumor model of human B-cell lymphoma OCI-LY10 cells**

**Objective:**

The antitumor effect of the test compound was evaluated using a SCID mouse xenograft tumor model of human B-cell lymphoma OCI-LY10 cells.

**Experimental materials:**

1. Experimental animals: SCID mice, female, 6 to 8 weeks old, weighing 17 to 20 g. Beijing Vital River Laboratory Animal Technology Co., Ltd.
2. Cell line: The human B-cell lymphoma OCI-LY10 cell line was purchased from Nanjing Cobioer Biosciences Co., Ltd., with catalog number CBP60558.

Table **22.** Reagent Information

| Name | Manufacturer | Batch Number | Storage Conditions |
|---|---|---|---|
| IMDM Medium | GIBCO, USA | 2323369 | 4°C |
| Fetal Bovine Serum (FBS) | GIBCO, USA | 2305262RP | -20°C |
| Matrigel (Matrigel) | CORNING | 2062001 | -20°C |

Table **23.** Instruments

| Name | Manufacturer | Model |
|---|---|---|
| SHJ Series Clean Bench | Shanghai Shangjing Purification Equipment Co., Ltd. | CA-1390-1 |
| $CO_2$ Water-Jacketed Cell Culture Incubator | Thermo Scientific Forma | 3111 |
| Inverted Microscope | Olympus | CKX41SF |
| Electric Suction Device | Shanghai Medical Instruments (Group) Co., Ltd. | YX930D |
| Eppendorf AG Centrifuge | Eppendorf | 5811XG639987 |
| Balance (0.01 g Precision) | Shanghai Minqiao Precise Science Instrument Co., Ltd. | SL502N |
| Balance (0.001 g Precision) | Shanghai Jinghai Instrument Co., Ltd. | JA2003N |
| Electronic Digital Caliper | SHAHE | (0 to 150) mm |

**Model establishment:**

OCI-LY10 cells were cultured in IMDM medium containing 20% FBS and maintained in a 5% $CO_2$ incubator at 37°C with saturated humidity. OCI-LY10 cells in the logarithmic growth phase were collected, resuspended in IMDM basal medium, mixed 1:1 with Matrigel, and adjusted to a cell concentration of $4\times 10^7$/mL. Under sterile conditions, 0.1 mL of the cell suspension was subcutaneously inoculated into the right back of SCID mice at an inoculation concentration of $4\times10^6$/0.1 mL/mouse.

**Experimental scheme:**

In pharmacodynamic experiments, when the tumors reached a certain size, animals with excessively large or

small tumor volumes or irregular tumor shapes were excluded. Animals with tumor volumes ranging from 167.65 to 231.29 $mm^3$ were selected and randomly grouped based on tumor volume, with 6 mice per group. The average tumor volume was approximately 201.15 $mm^3$. The day of grouping was designated as Day 0, and drug administration was initiated according to the animals' body weights. The pharmacodynamic experiment period was 28 days, with the drug administered once a day with a dosing interval of 24 hours, and administered intragastrically. During the experiment, animal body weight and tumor size were measured twice weekly. Clinical symptoms were observed and recorded daily.

The test compounds were administered at doses of 10 mg/kg, 30 mg/kg, and 100 mg/kg, with the vehicle being 10% DMSO/10% Solutol/80% $H_2O$. Tumor volume (TV) was calculated using the formula: $1/2 \times a \times b^2$, where a and b represent the measured length and width of the tumor, respectively. The tumor growth inhibition rate (TGI, %) was calculated using the formula: TGI (%) = [1 - (average tumor volume at the end of administration in a treatment group - average tumor volume at the beginning of administration in this treatment group)/(average tumor volume at the end of treatment in the vehicle control group - average tumor volume at the beginning of treatment in the vehicle control group)] x 100%. The relative tumor proliferation rate (T/C, %) was calculated using the formula: T/C % = $T_{RTV}$ / $C_{RTV}$ $\times$ 100% ($T_{RTV}$: mean RTV of the treatment group; $C_{RTV}$: mean RTV of the negative control group). The relative tumor volume (RTV) was calculated based on the tumor measurement results, with the formula RTV = $V_t/V_0$, where $V_0$ is the tumor volume measured at the time of grouping administration (*i.e.,* Day 0), and $V_t$ is the tumor volume at a certain measurement time. $T_{RTV}$ and $C_{RTV}$ were derived from data on the same day.

**Data analysis:**

In this study, experimental data were expressed as Mean±SEM.

Statistical analysis was performed using IBM SPSS Statistics software based on RTV data at the end of the experiment. Comparisons between two groups were analyzed by T test, while comparisons among three or more groups were analyzed by one-way ANOVA. If the variance was homogeneous (no significant difference in F-value), Tukey's method was applied for analysis. If the variance was heterogeneous (significant difference in F-value), the Games-Howell method was applied for testing. $p < 0.05$ was considered to be significantly different.

**Experimental results:** Test results are shown in Tables **24** and **25.**

Table **24.** Evaluation of the tumor-inhibitory efficacy of the compound of the present disclosure in a subcutaneous xenograft tumor model of human B-cell lymphoma OCI-LY 10 cells

| Group | Dosage | Tumor Volume ($mm^3$)[a] (Day 0) | Tumor Volume ($mm^3$)[a] (Day 28) | Relative Tumor Volume[a] (Day 28) | T/C (%) (Day 28) | TGI (%) (Day 28) |
|---|---|---|---|---|---|---|
| Vehicle Control | 0 mg/kg | 201.76 ± 7.08 | 1422.84 ± 89.14 | 7.11±0.56 | - | - |
| Hydrochloride of Compound of Formula (I) | 10 mg/kg | 202.43 ± 8.41 | 685.61 ± 75.95 | 3.37 ± 0.34 | 47.40 | 60.43 |
| Hydrochloride of Compound of Formula (I) | 30 mg/kg | 199.80 ± 8.02 | 322.68 ± 20.47 | 1.64 1 0.17 | 23.07 | 89.94 |
| Hydrochloride of Compound of Formula (I) | 100 mg/kg | 199.75 ± 7.96 | 188.01 ± 35.59 | 0.91 ± 0.15 | 12.80 | 100.96 |
| Note: a. Mean ± SEM. | | | | | | |

Table **25.** *p* values for comparison of relative tumor volumes among groups in the human B-cell lymphoma OCI-LY10 xenograft tumor model treated with the compound of the present disclosure

| Group | Vehicle Control | Hydrochloride of Compound of Formula (I) 10 mg/kg | Hydrochloride of Compound of Formula (I) 30 mg/kg | Hydrochloride of Compound of Formula (I) 100 mg/kg |
|---|---|---|---|---|
| Vehicle Control | N/A | 0.003 | 0.001 | <0.001 |

(continued)

| Group | Vehicle Control | Hydrochloride of Compound of Formula (I) 10 mg/kg | Hydrochloride of Compound of Formula (I) 30 mg/kg | Hydrochloride of Compound of Formula (I) 100 mg/kg |
|---|---|---|---|---|
| Hydrochloride of Compound of Formula (I) 10 mg/kg | 0.003 | N/A | 0.018 | 0.003 |
| Hydrochloride of Compound of Formula (I) 30 mg/kg | 0.001 | 0.018 | N/A | 0.077 |
| Hydrochloride of Compound of Formula (I) 100 mg/kg | <0.001 | 0.003 | 0.077 | N/A |
| Note: The *p* value was analyzed using IBM SPSS Statistics software. | | | | |

**Conclusion:** The compound of the present disclosure exhibits a significant tumor inhibitory effect in the SCID mouse xenograft tumor model of human B-cell lymphoma OCI-LY10 cells in a dose-dependent manner.

**Experimental Example 9: *In vivo* pharmacodynamic study in the CB17 SCID mouse subcutaneous xenograft tumor model of human lymphoma SU-DHL-2 cells**

**Objective:**

The antitumor effect of the test compound was evaluated using a SU-DHL-2 subcutaneous xenograft tumor model of CB17 SCID mice.

**Experimental materials:**

1. Experimental animals: CB17 SCID mice, female, 6 to 8 weeks old, weighing 18 to 22 g. Beijing Vital River Laboratory Animal Technology Co., Ltd.
2. Cell line: Human lymphoma SU-DHL-2 cells (Cat. No.: ATCC-CRL-2956).

Table **26.** Reagent Information

| Name | Manufacturer | Cat. No. | Batch Number | Expiration Date | Storage Conditions |
|---|---|---|---|---|---|
| 1640 Medium | Gibco | 22400089 | 2462009 | 2023-02-28 | 4°C |
| Matrigel | Corning | 354234 | 2013002 | 2022-10-19 | -20°C |
| 100× Double Antibiotics (Penicillin, Streptomycin) | Meilunbio | MA0110 | MA0110-Apr-15H | 2023-04-14 | -20°C |
| PBS | HyClone | SH30256.01 | AG29791799 | 2023-08-31 | 4°C |
| Fetal Bovine Serum | ExCell Bio | FND500 | 11H233 | 2023-08 | -20°C |

Table **27.** Instruments

| Name | Manufacturer | Model |
|---|---|---|
| Biosafety Cabinet | Suzhou Antai Airtech Co., Ltd. | BSC-1604IIA2 |
| Vernier Caliper | Mitutoyo | CD-6"ASX |

(continued)

| Name | Manufacturer | Model |
|---|---|---|
| Electronic Balance | Changzhou Tianzhiping Instruments Co., Ltd. | EL-2KJ |
| Vortex Vibrator | HaiMen City Qilin Medical Instrument Factory | XW-80A |
| Pure Water System | Millipore | MILLI-Q®Direct8 |

**Model establishment:**

Cell culture: Human lymphoma SU-DHL-2 cells (ATCC-CRL-2956) were cultured in suspension *in vitro* under conditions of RPMI 1640 medium supplemented with 10% inactivated fetal bovine serum, 100 U/mL penicillin, and 100 μg/mL streptomycin, and incubated at 37°C in a 5% $CO_2$ incubator. Routine passaging was performed twice a week. When the cell saturation density was 80% to 90% and the number of cells reached the required level, the cells were collected, counted, and inoculated.

Tumor cell inoculation and grouping: 0.2 mL ($10\times10^6$ cells) (PBS: matrigel = 1:1) of SU-DHL-2 cells were subcutaneously inoculated into the right back of each mouse. When the average tumor volume reached approximately 139 $mm^3$, the drugs were administered in groups. The day of grouping was designated as Day 0, and drug administration was initiated according to the animals' body weights.

**Experimental scheme:**

The pharmacodynamic experiment was conducted with a 7-day administration cycle, where the test compound was administered once daily intragastrically at 24-hour intervals, for a total of three cycles. During the experiment, animal body weight and tumor size were measured twice a week, and clinical symptoms were observed and recorded daily.

The test compounds were administered at doses of 10 mg/kg, 30 mg/kg, and 100 mg/kg, with the vehicle being 10% DMSO/10% Solutol/80% $H_2O$. Tumor volume (TV) was calculated using the formula: $1/2 \times a \times b^2$, where a and b represent the measured length and width of the tumor, respectively. The tumor growth inhibition rate (TGI, %) was calculated using the formula: TGI (%) = [1 - (average tumor volume at the end of administration in a treatment group - average tumor volume at the beginning of administration in this treatment group))/(average tumor volume at the end of treatment in the vehicle control group - average tumor volume at the beginning of treatment in the vehicle control group)] x 100%. The relative tumor proliferation rate (T/C, %) was calculated using the formula: T/C % = $T_{RTV/RTV}\times$ 100% ($T_{RTV}$: mean RTV of the treatment group; $C_{RTV}$: mean RTV of the negative control group). The relative tumor volume (RTV) was calculated based on the tumor measurement results, with the formula RTV = $V_t/V_0$, where $V_0$ is the tumor volume measured at the time of grouping administration (*i.e.,* Day 0), and $V_t$ is the tumor volume at a certain measurement time. $T_{RTV}$ and $C_{RTV}$ were derived from data on the same day.

**Data analysis:**

Statistical analysis was performed using SPSS software based on RTV data at the end of the experiment. Comparisons between two groups were analyzed by T test, while comparisons among three or more groups were analyzed by one-way ANOVA. If the variance was homogeneous (no significant difference in F-value), Tukey's method was applied for analysis. If the variance was heterogeneous (significant difference in F-value), the Games-Howell method was applied for testing. $p < 0.05$ was considered to be significantly different.

**Experimental results:** Test results are shown in Tables **28** and **29.**

Table **28.** Evaluation of the tumor-inhibitory efficacy of the compound of the present disclosure in the subcutaneous xenograft tumor model of lymphoma SU-DHL-2 cells

| Group | Dosage | Tumor Volume ($mm^3$)[a] (Day 0) | Tumor Volume ($mm^3$)[a] (Day 21) | Relative Tumor Volume[a] (Day 21) | T/C (%) (Day 21) | TGI (%) (Day 21) |
|---|---|---|---|---|---|---|
| Vehicle Control | 0 mg/kg | 139 ± 6 | 1883 ± 141 | 13.68 | - | - |

(continued)

| Group | Dosage | Tumor Volume ($mm^3$)[a] (Day 0) | Tumor Volume ($mm^3$)[a] (Day 21) | Relative Tumor Volume[a] (Day 21) | T/C (%) (Day 21) | TGI (%) (Day 21) |
|---|---|---|---|---|---|---|
| Hydrochloride of Compound of Formula (I) | 10 mg/kg | 139 ± 6 | 800 + 48 | 5.79 | 42.33 | 62.13 |
| | 30 mg/kg | 139 ± 6 | **813** ± 99 | 5.78 | 42.26 | 61.41 |
| | 100 mg/kg | 139 ± 5 | 626 ± 51 | 4.55 | 33.23 | 72.07 |
| **[0287]** Note: a. Mean ± SEM. | | | | | | |

Table **29.** *p* values for comparison of relative tumor volumes among groups in the lymphoma SU-DHL-2 xenograft tumor model treated with the compound of the present disclosure

| Group | Vehicle Control | Hydrochloride of Compound of Formula (I) 10 mg/kg | Hydrochloride of Compound of Formula (I) 30 mg/kg | Hydrochloride of Compound of Formula (I) 100 mg/kg |
|---|---|---|---|---|
| Vehicle Control | N/A | 0.001 | 0.001 | <0.001 |
| Hydrochloride of Compound of Formula (I) 10 mg/kg | 0.001 | N/A | 1.000 | 0.268 |
| Hydrochloride of Compound of Formula (I) 30 mg/kg | 0.001 | 1.000 | N/A | 0.507 |
| Hydrochloride of Compound of Formula (I) 100 mg/kg | <0.001 | 0.268 | 0.507 | N/A |
| Note: The *p* value was obtained by analyzing the relative tumor volume (RTV) using one-way ANOVA. Data with heterogeneous variance were analyzed using Games-Howell. | | | | |

**Conclusion:** The compound of the present disclosure exhibits a significant tumor inhibitory effect in a CB17 SCID mouse subcutaneous xenograft tumor model of human lymphoma SU-DHL-2 cells.

**Experimental Example 10: *In vivo* pharmacodynamic study on a BALB/c nude mouse subcutaneous xenograft tumor model of human diffuse large B-cell lymphoma TMD-8 cells**

**Objective:**

The antitumor effect of the test compound was evaluated using a BALB/c nude mouse subcutaneous xenograft tumor model of human diffuse large B-cell lymphoma TMD-8 cells.

**Experimental materials:**

1. Experimental animals: BALB/c nude mice, female, 6 to 8 weeks old. Vital River Laboratory Animal Technology Co., Ltd.
2. Cell line: Human diffuse large B-cell lymphoma TMD-8 cells (purchased from Shanghai Huzhen Industrial Co., Ltd.).

Table **30.** Main Reagent Information

| **Name** | **Manufacturer** | **Cat. No.** | **Batch Number** |
|---|---|---|---|
| RPMI-1640 Powder | Gibco | 31800-022 | 2383858 |

(continued)

| Name | Manufacturer | Cat. No. | Batch Number |
|---|---|---|---|
| Fetal Bovine Serum (FBS) | Gibco | 10099-141C | 2217479CP |
| Matrigel | Corning | 354248 | 2139002 |
| PBS | Beyotime | ST447 | 100921220308 |
| Double Antibiotics | Hyclone | SV30010 | J210026 |
| DMSO | Aladdin | D103273 | H2120209 |
| Solutol HS15 | BASF | 50379938 | 42608009T0 |
| Sterile Water for Injection | Zhejiang Dubang Pharmaceutical | NA | 2111290103 |

Table **31.** Main Instrument Information

| Name | Manufacturer | Model |
|---|---|---|
| Vernier Caliper | Mitutoyo Corporation | CD-6"CX |
| Ultrasonic Homogenizer | Kunshan Ultrasonic Instruments Co., Ltd. | KQ-700DE |
| Balance | METTLER TOLEDO | XP 105 |
| Vortex Mixer | Thermolyne | 16700 |

**Model establishment:**

Cell culture: Routine cell culture was conducted in RPMI-1640 medium containing 10% fetal bovine serum under 5% $CO_2$ at 37°C. Cells were passaged based on cell growth conditions at a passage ratio of 1:3 to 1:4.

Tumor cell inoculation and grouping: TMD-8 cells in the logarithmic growth phase were harvested, counted, and resuspended in a mixture of 50% serum-free RPMI-1640 medium and 50% Matrigel. The cell concentration was adjusted to 4.0x$10^7$ cells/mL. The cell suspension was placed in an ice box, aspirated using a 1-mL syringe, and injected subcutaneously into the right forelimb axilla of nude mice at 200 μLper animal ($0.8\times10^7$ cells/mouse) to establish the TMD-8 xenograft tumor model. Administration was started when the average tumor volume reached approximately 160 $mm^3$. The day of grouping was designated as Day 1 (D1) of the experiment, and administration was started based on animal body weight.

**Experimental scheme:**

The pharmacodynamic experiment was conducted with a 7-day administration cycle, where the test compound was administered once daily intragastrically at 24-hour intervals, for a total of three cycles. During the experiment, animal body weight and tumor size were measured twice a week, and clinical symptoms were observed and recorded daily.

The test compound was administered at doses of 10 mg/kg, 30 mg/kg, and 100 mg/kg, with the vehicle being 10% DMSO/10% Solutol HS15/80% water. Tumor volume (TV) was calculated using the formula: $1/2 \times a \times b^2$, where a and b represent the measured length and width of the tumor, respectively. The tumor growth inhibition rate (TGI, %) was calculated using the formula: TGI (%) = [1 - (average tumor volume at the end of administration in a treatment group - average tumor volume at the beginning of administration in this treatment group)/(average tumor volume at the end of treatment in the vehicle control group - average tumor volume at the beginning of treatment in the vehicle control group)] x 100%. The relative tumor proliferation rate (T/C, %) was calculated using the formula: T/C % = $T_{RTV}/C_{RTV}$x 100% ($T_{RTV}$: mean RTV of the treatment group; $C_{RTV}$: mean RTV of the negative control group). The relative tumor volume (RTV) was calculated based on the tumor measurement results, with the formula RTV = $V_t/V_0$, where $V_0$ is the tumor volume measured at the time of grouping administration (*i.e.,* Day 1), and $V_t$ is the tumor volume at a certain measurement time. $T_{RTV}$ and $C_{RTV}$ were derived from data on the same day.

**Data analysis:**

The experimental data were calculated and subjected to relevant statistical processing using Microsoft Office Excel 2007 software. Unless otherwise specified, the data were expressed as mean ± standard error (Mean ± SE), and comparisons between groups were performed using t-tests.

**Experimental results:** The test results are shown in Table **32.**

Table **32.** Evaluation of the tumor-inhibitory efficacy of the compound of the present disclosure in the nude mouse xenograft tumor model of human diffuse large B-cell lymphoma TMD-8 cells

| Group | Dosage | Tumor Volume ($mm^3$)[a] (Day 0) | Tumor Volume ($mm^3$)[a] (Day 22) | Relative Tumor Volume[a] (Day 22) | T/C (%) (Day 22) | TGI (%) (Day 22) |
|---|---|---|---|---|---|---|
| Vehicle Control | 0 mg/kg | 160 ± 10 | 1234 ± 179 | 7.62 | - | - |
| Hydrochloride of Compound of Formula (I) | 10 mg/kg | **161** ± 12 | 714 ± 153* | 4.41 | 58 | 49 |
| | 30 mg/kg | 160 ± 9 | 430 ± 98** | 2.60 | 34 | 75 |
| | 100 mg/kg | 160 ± 8 | 291 ± 70** | 1.78 | 23 | 88 |
| Note: "*" indicates a significant difference in tumor volume compared to the vehicle control group ($P$ < 0.05); "**" indicates a highly significant difference compared to the vehicle control group ($P$ < 0.01); "[a]" indicates mean ± SEM. | | | | | | |

**Conclusion:** The compound of the present disclosure exhibits a significant tumor inhibitory effect in the BALB/c nude mouse subcutaneous xenograft tumor model of human diffuse large B-cell lymphoma TMD-8 cells.

## Claims

1. A crystal form A of a compound of formula (I),

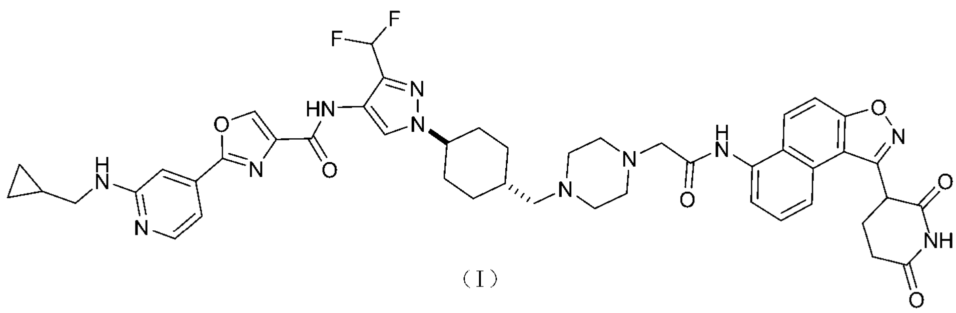

(I)

,

wherein the crystal form A of the compound of formula (I) has an X-ray powder diffraction pattern comprising characteristic diffraction peaks at the following 2θ angles: 8.91±0.20°, 11.15±0.20', 17.26±0.20°, 21.14±0.20°, and 23.38±0.20°.

2. The crystal form A of the compound of formula (I) according to claim 1, wherein the X-ray powder diffraction pattern thereof comprises characteristic diffraction peaks at 6, 7, or 8 of the following 20 angles: 5.02±0.20°, 8.91±0.20°, 11.15+0.20°, 15.08±0.20°, 17.26±0.20°,18.3810.20°, 21.1410.20°, 23.3810.20°, and 23.574±0.20°.

3. The crystal form A of the compound of formula (I) according to claim 1, wherein the X-ray powder diffraction pattern thereof comprises characteristic diffraction peaks at the following 2θ angles: 5.02±0.20°, 8.91±0.20°, 11.15+0.20°, 15.08±0.20°, 17.26±0.20°, 18.38±0.20°, 21.1410.20°, and 23.57±0.20°.

4. The crystal form A of the compound of formula (I) according to claim 1, wherein the X-ray powder diffraction pattern thereof comprises characteristic diffraction peaks at 10, 11, 12, or 13 of the following 20 angles: 5.02±0.20°, 8.91 ±0.20°, 9.80±.20°, 11.15+0.20°, 13.87±0.20°, 14.70±0.20°, 15.08±0.20°, 17.26±0.20°, 18.38±0.20° 21.14 ±0.20°, 21.57±0.20°, 23.10±0.20°, 23.38±.20°, and 23.57±0.20°.

5. The crystal form A of the compound of formula (I) according to claim 1, wherein the X-ray powder diffraction pattern thereof comprises characteristic diffraction peaks at the following 2θ angles: 5.02±0.20°, 8.91±0.20°, 9.80±.20°, 11.15±0.20°, 13.87±0.20°, 14.70±0.20°, 15.08±0.20°, 17.26±0.20°,18.38±0.20° 21.14±0.20°, 21.57±0.20°, and 23.38±0.20°.

**6.** The crystal form A of the compound of formula (I) according to claim 1, wherein the X-ray powder diffraction pattern thereof comprises characteristic diffraction peaks at the following 2θ angles: 5.02±0.20°, 8.91±0.20°, 9.80±.20°, 11.15±0.20°, 12.72±0.20°, 13.87±0.20°, 14.70±0.20°, 15.08±0.20°, 16.41±0.20°, 17.26±0.20°, 18.38±0.20°, 19.22±0.20°, 19.86±0.20°, 21.14±0.20°, 21.57±0.20°, 22.29±0.20°, 23.10±0.20°, 23.3810.20°, 23.5710.20°, 25.71±0.20°, 26.49±0.20°, and 28.91±0.20°.

**7.** The crystal form A of the compound of formula (I) according to claim 1, wherein the X-ray powder diffraction pattern thereof comprises characteristic diffraction peaks at the following 20 angles: 5.02°, 8.91°, 9.80°, 11.15°, 12.72°, 13.87°, 14.70°, 15.08°, 16.41°, 17.26°, 18.38°, 19.22°, 19.86°, 21.14°, 21.57°, 22.29°, 23.10°, 23.38°, 23.57°, 25.71°, 26.49°, and 28.91°.

**8.** The crystal form A of the compound of formula (I) according to claim 1, wherein the X-ray powder diffraction pattern of the crystal form A is substantially as shown in FIG. 1.

**9.** The crystal form A of the compound of formula (I) according to claim 1, wherein the crystal form A has a differential scanning calorimetry curve showing an endothermic peak with a peak temperature of 272.8°C±3°C and an exothermic peak with a peak temperature of 275.4°C±3°C.

**10.** The crystal form A of the compound of formula (I) according to claim 1, wherein the crystal form A of the compound of formula (I) has a differential scanning calorimetry curve substantially as shown in FIG. 2.

**11.** The crystal form A of the compound of formula (I) according to claim 1, wherein the crystal form A has a thermogravimetric analysis curve showing a weight loss of 1.00% at 150.0°C±3.0°C.

**12.** The crystal form A of the compound of formula (I) according to claim 1, wherein the crystal form A of the compound of formula (I) has a thermogravimetric analysis curve substantially as shown in FIG. 3.

**13.** Use of the crystal form A of the compound of formula (I) according to any one of claims 1 to 12 in the manufacture of a medicament for treating diffuse large B-cell lymphoma.

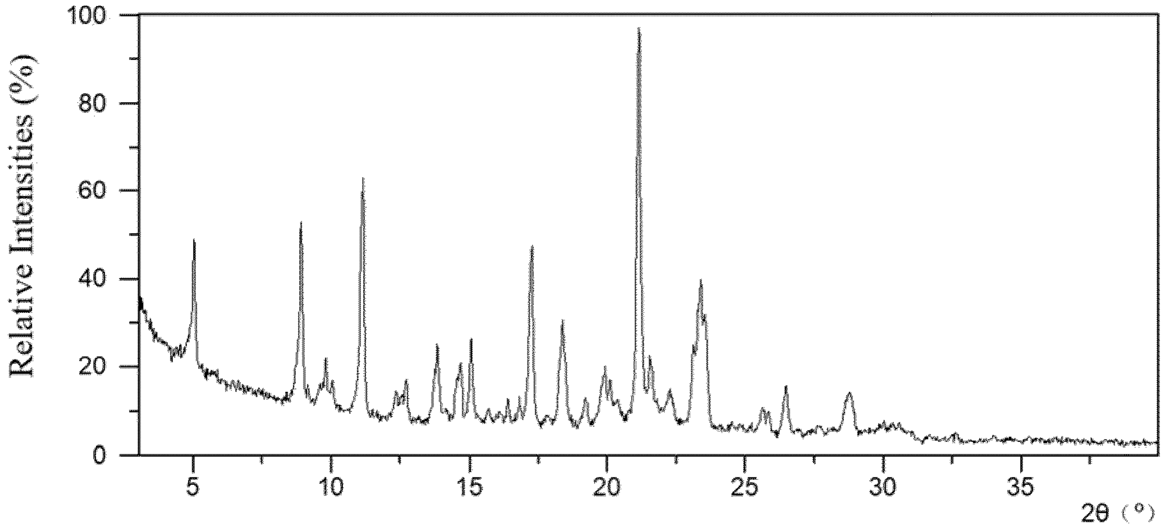
100
80
60
40
20
0
Relative Intensities (%)
5
10
15
20
25
30
35
2θ（°）

FIG. 1

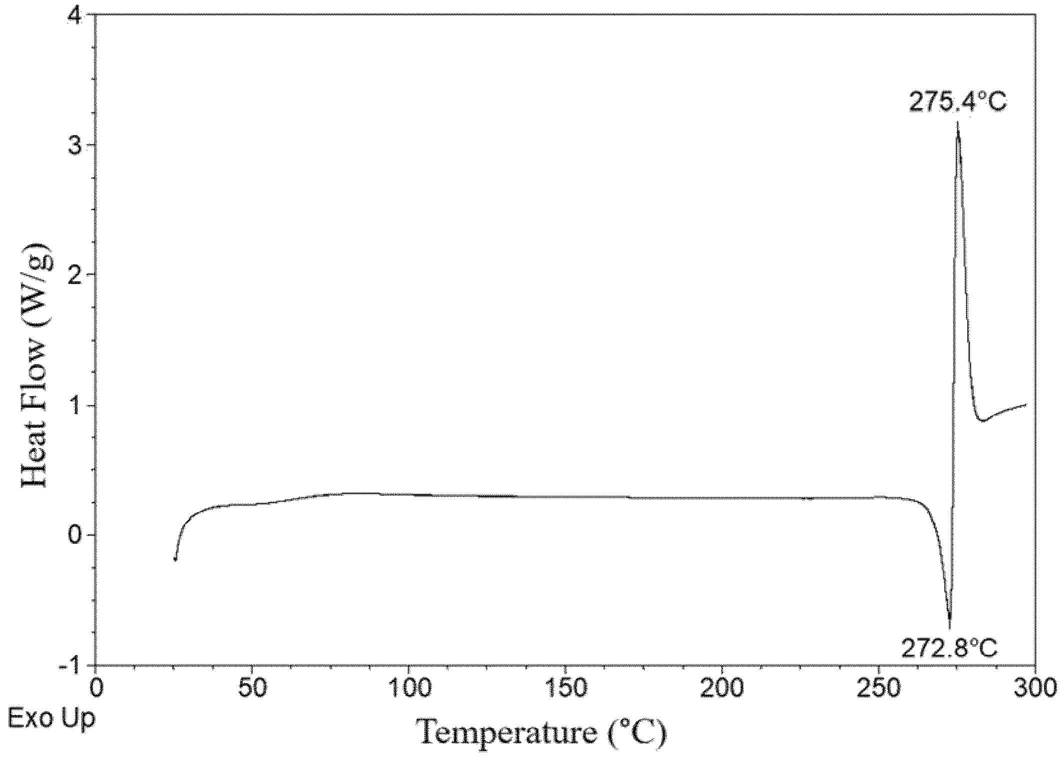
4
3
2
1
0
-1
Heat Flow (W/g)
275.4°C
272.8°C
0
50
100
150
200
250
300
Exo Up
Temperature (°C)

FIG. 2

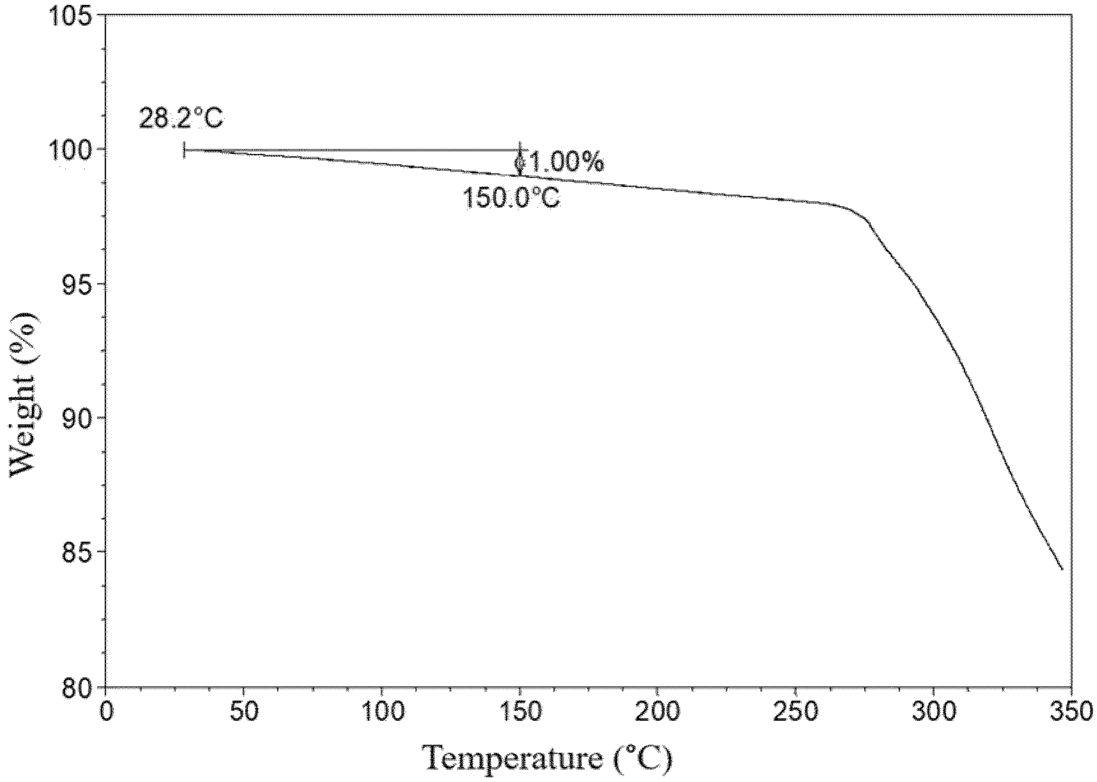
105
100
95
90
85
80
0
50
100
150
200
250
300
350
28.2°C
1.00%
150.0°C
Weight (%)
Temperature (°C)

FIG. 3

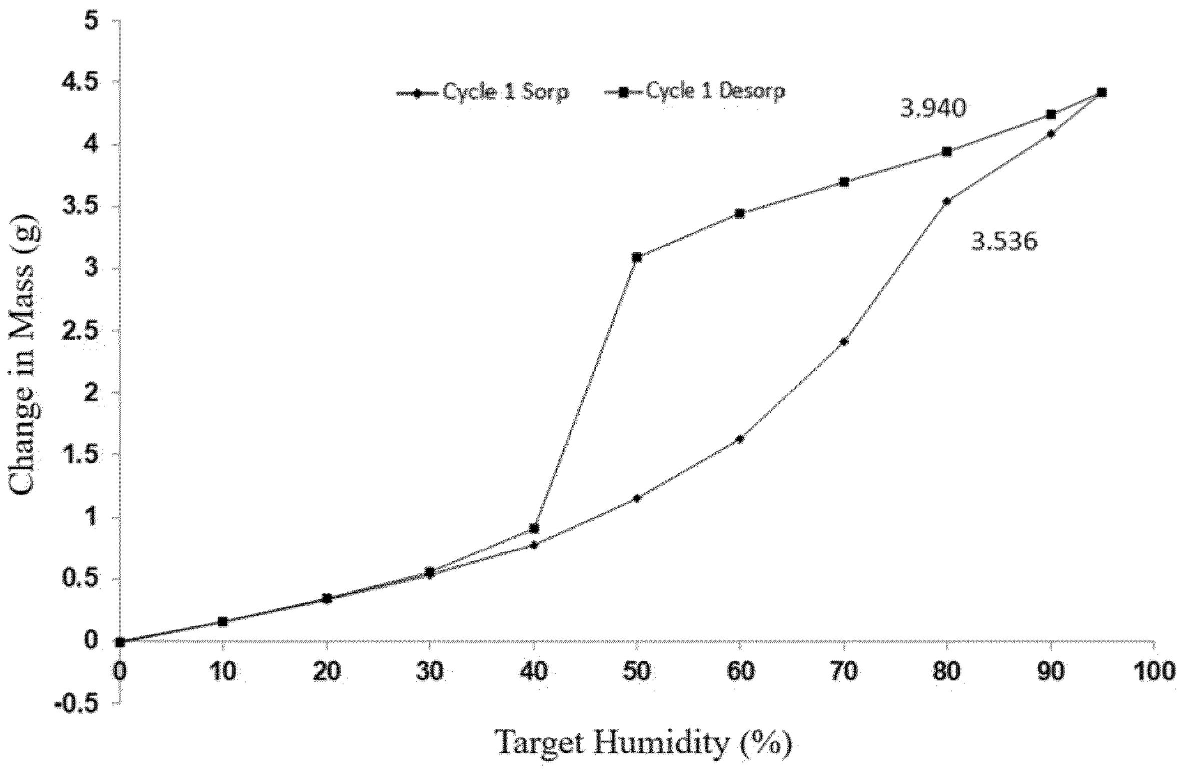
5
4.5
4
3.5
3
2.5
2
1.5
1
0.5
0
-0.5
0
10
20
30
40
50
60
70
80
90
100
Cycle 1 Sorp
Cycle 1 Desorp
3.940
3.536
Change in Mass (g)
Target Humidity (%)

FIG. 4

## INTERNATIONAL SEARCH REPORT

International application No.
**PCT/CN2024/083385**

**A. CLASSIFICATION OF SUBJECT MATTER**

C07D 413/14(2006.01)i; A61K31/4545(2006.01)i; A61K31/496(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C07D 413/-,A61K,A61P

Documentation searched other than minimum documentation to the extent that such documents are included in the fields searched

Electronic data base consulted during the international search (name of data base and, where practicable, search terms used)

CNKI, CNABS, CNTXT, ENTXT, DWPI, REGISTRY, CAPLUS, MARPAT: 2, 6-哌啶二酮, 白细胞介素, 蛋白降解靶向嵌合体, 肿瘤, 癌症, piperidinedion, IRAK4, PROTAC, CRBN, cancer, tumor, 根据权利要求1进行的结构式检索, structural formula search conducted according to claim 1.

**C. DOCUMENTS CONSIDERED TO BE RELEVANT**

| Category* | Citation of document, with indication, where appropriate, of the relevant passages | Relevant to claim No. |
|---|---|---|
| PX | WO 2023045978 A1 (MEDSHINE DISCOVERY INC.) 30 March 2023 (2023-03-30)<br>entire document | 1-13 |
| A | WO 2020264499 A1 (KYMERA THERAPEUTICS, INC.) 30 December 2020 (2020-12-30)<br>entire document, in particular, see claims 19-26, and table 1 | 1-13 |
| A | WO 2021127283 A2 (KYMERA THERAPEUTICS, INC.) 24 June 2021 (2021-06-24)<br>entire document, in particular, claims 1, 4 and 25-28, description, paragraph [0014], and table 1 | 1-13 |
| A | CN 113423427 A (KYMERA THERAPEUTICS, INC.) 21 September 2021 (2021-09-21)<br>entire document, in particular, claims 1, 3 and 20-26, and description, table 1 | 1-13 |
| A | CN 112105385 A (KYMERA THERAPEUTICS, INC.) 18 December 2020 (2020-12-18)<br>entire document | 1-13 |
| A | WO 2021127190 A1 (KYMERA THERAPEUTICS, INC.) 24 June 2021 (2021-06-24)<br>entire document | 1-13 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **18 June 2024** | **03 July 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/083385**

**C. DOCUMENTS CONSIDERED TO BE RELEVANT**

| Category* | Citation of document, with indication, where appropriate, of the relevant passages | Relevant to claim No. |
|---|---|---|
| A | WO 2021185291 A1 (MEDSHINE DISCOVERY INC.) 23 September 2021 (2021-09-23)<br>entire document | 1-13 |

Form PCT/ISA/210 (second sheet) (July 2022)

**INTERNATIONAL SEARCH REPORT**
**Information on patent family members**

International application No.
**PCT/CN2024/083385**

| Patent document cited in search report | | | Publication date (day/month/year) | Patent family member(s) | | | Publication date (day/month/year) |
|---|---|---|---|---|---|---|---|
| WO | 2023045978 | A1 | 30 March 2023 | CN | 118055929 | A | 17 May 2024 |
| WO | 2020264499 | A1 | 30 December 2020 | EP | 3989966 | A1 | 04 May 2022 |
| | | | | EP | 3989966 | A4 | 27 September 2023 |
| | | | | JP | 2022538192 | A | 31 August 2022 |
| | | | | US | 2023069104 | A1 | 02 March 2023 |
| | | | | BR | 112021026517 | A2 | 10 May 2022 |
| | | | | CO | 2021017893 | A2 | 17 January 2022 |
| | | | | CA | 3144805 | A1 | 30 December 2020 |
| | | | | KR | 20220032063 | A | 15 March 2022 |
| | | | | IL | 289267 | A | 01 February 2022 |
| | | | | MX | 2021015995 | A | 11 March 2022 |
| | | | | AU | 2020302118 | A1 | 24 February 2022 |
| | | | | CN | 114502158 | A | 13 May 2022 |
| WO | 2021127283 | A2 | 24 June 2021 | US | 2023234953 | A1 | 27 July 2023 |
| | | | | WO | 2021127283 | A3 | 29 July 2021 |
| | | | | US | 2022340570 | A1 | 27 October 2022 |
| | | | | US | 11591332 | B2 | 28 February 2023 |
| | | | | EP | 4076524 | A2 | 26 October 2022 |
| | | | | EP | 4076524 | A4 | 29 November 2023 |
| CN | 113423427 | A | 21 September 2021 | JP | 2022516401 | A | 28 February 2022 |
| | | | | BR | 112021010484 | A2 | 24 August 2021 |
| | | | | CO | 2021007068 | A2 | 30 September 2021 |
| | | | | IL | 283471 | A | 29 July 2021 |
| | | | | CA | 3119773 | A1 | 04 June 2020 |
| | | | | US | 2021323952 | A1 | 21 October 2021 |
| | | | | US | 11352350 | B2 | 07 June 2022 |
| | | | | EP | 3886904 | A1 | 06 October 2021 |
| | | | | EP | 3886904 | A4 | 13 July 2022 |
| | | | | KR | 20210111252 | A | 10 September 2021 |
| | | | | MX | 2021006154 | A | 24 August 2021 |
| | | | | AU | 2019389174 | A1 | 01 July 2021 |
| | | | | US | 2022324854 | A1 | 13 October 2022 |
| | | | | US | 11807636 | B2 | 07 November 2023 |
| | | | | WO | 2020113233 | A1 | 04 June 2020 |
| | | | | US | 11117889 | B1 | 14 September 2021 |
| | | | | SG | 11202105424 | PA | 29 June 2021 |
| CN | 112105385 | A | 18 December 2020 | IL | 304055 | A | 01 August 2023 |
| | | | | AU | 2018396142 | A1 | 16 July 2020 |
| | | | | US | 2023106066 | A1 | 06 April 2023 |
| | | | | US | 11723980 | B2 | 15 August 2023 |
| | | | | JP | 2024038329 | A | 19 March 2024 |
| | | | | SG | 11202005912 | PA | 29 July 2020 |
| | | | | US | 2019192668 | A1 | 27 June 2019 |
| | | | | US | 10874743 | B2 | 29 December 2020 |
| | | | | BR | 112020012997 | A2 | 01 December 2020 |
| | | | | MX | 2020006812 | A | 06 November 2020 |
| | | | | CA | 3086763 | A1 | 04 July 2019 |
| | | | | JP | 2021508703 | A | 11 March 2021 |
| | | | | EP | 3731869 | A1 | 04 November 2020 |
| | | | | EP | 3731869 | A4 | 22 September 2021 |

Form PCT/ISA/210 (patent family annex) (July 2022)

**INTERNATIONAL SEARCH REPORT**
**Information on patent family members**

International application No.
**PCT/CN2024/083385**

| Patent document cited in search report | Publication date (day/month/year) | Patent family member(s) | Publication date (day/month/year) |
|---|---|---|---|
| | | US 2023398223 A1 | 14 December 2023 |
| | | WO 2019133531 A1 | 04 July 2019 |
| | | IL 275649 A | 31 August 2020 |
| | | IL 275649 B1 | 01 August 2023 |
| | | IL 275649 B2 | 01 December 2023 |
| | | US 11318205 B1 | 03 May 2022 |
| WO 2021127190 A1 | 24 June 2021 | BR 112022011651 A2 | 23 August 2022 |
| | | US 2024131016 A1 | 25 April 2024 |
| | | MX 2022007576 A | 23 September 2022 |
| | | AU 2020407200 A1 | 21 July 2022 |
| | | EP 4076520 A1 | 26 October 2022 |
| | | EP 4076520 A4 | 27 March 2024 |
| | | CO 2022008406 A2 | 08 July 2022 |
| | | CA 3161878 A1 | 24 June 2021 |
| | | TW 202136251 A | 01 October 2021 |
| | | IL 293917 A | 01 August 2022 |
| | | KR 20220145325 A | 28 October 2022 |
| | | JP 2023509366 A | 08 March 2023 |
| | | US 2023144292 A1 | 11 May 2023 |
| | | US 11707457 B2 | 25 July 2023 |
| | | US 2021228562 A1 | 29 July 2021 |
| | | US 11779578 B2 | 10 October 2023 |
| | | CN 115052627 A | 13 September 2022 |
| WO 2021185291 A1 | 23 September 2021 | US 2023158152 A1 | 25 May 2023 |
| | | EP 4122925 A1 | 25 January 2023 |
| | | EP 4122925 A4 | 17 April 2024 |
| | | JP 2023517393 A | 25 April 2023 |
| | | CN 115380026 B | 22 November 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2023102936424